(19) Europäisches Patentamt
European Patent Office
Office européen des brevets

(11) **EP 4 620 538 A1**

(12) **EUROPEAN PATENT APPLICATION**
published in accordance with Art. 153(4) EPC

(43) Date of publication:
**24.09.2025 Bulletin 2025/39**

(21) Application number: **24777459.9**

(22) Date of filing: **18.01.2024**

(51) International Patent Classification (IPC):
**A63B 24/00** (2006.01)     **G06F 16/9035** (2019.01)

(52) Cooperative Patent Classification (CPC):
**A63B 24/00; G06F 3/01; G06F 16/9035**

(86) International application number:
**PCT/CN2024/073070**

(87) International publication number:
**WO 2024/198664 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.03.2023 CN 202310377258**

(71) Applicant: **Huawei Technologies Co., Ltd.
Shenzhen, Guangdong 518129 (CN)**

(72) Inventors:
• **TI, Jian
  Shenzhen, Guangdong 518129 (CN)**
• **JIANG, Jin
  Shenzhen, Guangdong 518129 (CN)**
• **XIONG, Chao
  Shenzhen, Guangdong 518129 (CN)**

(74) Representative: **Goddar, Heinz J.
Boehmert & Boehmert
Anwaltspartnerschaft mbB
Pettenkoferstrasse 22
80336 München (DE)**

(54) **EXERCISE CONTROL METHOD, ELECTRONIC DEVICE, AND COMPUTER READABLE MEDIUM**

(57) This application relates to the field of terminal technologies, and specifically, to an exercise control method, an electronic device, and a computer-readable medium. The method includes: obtaining an exercise target parameter of a user for an exercise apparatus; determining a first training plan of the user based on the exercise target parameter and a historical exercise capability parameter of the user, where the historical exercise capability parameter includes a physical function parameter generated in a process in which the user takes exercise by using the exercise apparatus; and sending a first set of control instructions to the exercise apparatus based on the first training plan. **In** this way, a training plan that meets a training target can be generated with reference to an actual exercise capability determined based on a historical exercise record of the user, to help improve an actual exercise effect and exercise safety of the user.

FIG. 3

**Description**

This application claims priority to Chinese Patent Application No. 202310377258.2, filed with the China National Intellectual Property Administration on March 31, 2023 and entitled "EXERCISE CONTROL METHOD, ELECTRONIC DEVICE, AND COMPUTER-READABLE MEDIUM", which is incorporated herein by reference in its entirety.

**TECHNICAL FIELD**

The present invention relates to the field of terminal technologies, and specifically, to an exercise control method, an electronic device, and a computer-readable medium.

**BACKGROUND**

With gradual improvement of health awareness of users, a consumer market for sports devices is gradually expanding. Consumers are inclined to select more professional sports venues and use more professional exercise apparatuses for exercise, such as treadmills, spin bikes, and elliptical trainers. In addition, as intelligentization of these exercise apparatuses is continuously improved, some exercise apparatuses can be connected to a mobile device or a wearable device used by a user, to monitor an exercise process. The user not only may set an exercise target, such as a distance target, a time target, and a calorie burning target, on the exercise apparatus, but also may intuitively view a heart rate change and the like of the user in the exercise process. In this way, the user can complete a specified target as planned, to achieve an expected training objective.

Therefore, in a process in which an existing exercise apparatus assists in exercise, to improve exercise safety, currently, there are also some cases in which physiological data of the user is monitored in real time, for example, a real-time heart rate is monitored by a wearable device such as a watch or a headset worn by the user, to guide the user to achieve a training objective more safely and efficiently. It may be understood that the physiological data of the user can represent, to some extent, current exercise intensity achieved by the user, and with reference to a cardiopulmonary endurance range of the user, the exercise intensity of the user can be controlled within a safe range. For example, in a process in which the user takes exercise by using an exercise apparatus, based on a monitored real-time heart rate of the user, a corresponding load of the exercise apparatus may be adjusted to match cardiopulmonary endurance of the user.

However, in a current exercise control solution, in combination with a real-time heart rate of the user, the load of the exercise apparatus can be adjusted based on only an apparatus parameter that is set by the user, for example, an initial speed that is set by the user on a treadmill. In addition, the apparatus parameter that is set by the user does not necessarily match an actual exercise capability of the user. In this case, if the initial speed, resistance intensity, or the like of the exercise apparatus is high or low, an adjustment amplitude and an adjustment frequency cannot be controlled according to the solution of adjusting the load based on the heart rate of the user. Frequent or large-scale adjustment of the load of the exercise apparatus is likely to cause discomfort of the user, resulting in poor exercise experience of the user.

**SUMMARY**

This application provides an exercise control method, an electronic device, and a computer-readable medium, to help improve an actual exercise effect and exercise safety. Further, a generated training plan can be dynamically adjusted based on an actual exercise status of a user in an exercise process of the user, to ensure safety of the user in the exercise process and bring better exercise experience to the user.

According to a first aspect, this application provides an exercise control method, applied to a first electronic device. The method includes:
obtaining an exercise target parameter of a user for an exercise apparatus; determining a first training plan of the user based on the exercise target parameter and a historical exercise capability parameter of the user, where the historical exercise capability parameter includes a physical function parameter generated in a process in which the user takes exercise by using the exercise apparatus; and sending a first set of control instructions to the exercise apparatus based on the first training plan.

For example, the first training plan is a training plan generated based on a training target input by the user and an actual exercise capability. The training target input by the user includes the foregoing exercise target parameter for the exercise apparatus, and may be, for example, a distance target, a time target, or a calorie consumption target. The historical exercise capability parameter of the user may be a related parameter that can indicate the actual exercise capability of the user and that is obtained through training or statistics collection based on a historical exercise record of by the user, namely, the foregoing physical function parameter, for example, a maximum speed interval that the user can bear, an incline interval frequently used by the user, or a maximum power interval of a resistance-type apparatus that the user can bear. This is not limited herein.

The first electronic device may be an electronic device such as a mobile phone, and the first set of control instructions may be one or more control instructions sent by the mobile phone or the like to the exercise apparatus. The control instructions may correspond to action phases in the first training plan respectively, and instruct the exercise apparatus to adjust, based on a corresponding training plan, a load corresponding to each action phase.

According to the solution provided in the first aspect, the training plan that meets the training target can be generated with reference to the actual exercise capability determined based on the historical exercise record of the user, to help improve an actual exercise effect and exercise safety of the user.

In a possible implementation of the first aspect, the first training plan includes at least one action phase, and an exercise control parameter corresponding to each action phase matches the historical exercise capability parameter of the user.

In a possible implementation of the first aspect, the exercise control parameter corresponding to each action phase in the first training plan includes a load parameter related to the exercise apparatus; and sending the first control instruction to the exercise apparatus based on the first training plan includes:

sending a first control instruction for a first action phase to the exercise apparatus based on a first load parameter corresponding to the first action phase, where the first control instruction includes the first load parameter, and the first set of control instructions include the first control instruction.

For example, the load parameter related to the exercise apparatus may be a speed parameter or an incline parameter corresponding to the exercise apparatus such as a treadmill, or may be a power parameter or the like corresponding to the exercise apparatus such as a spin bike. This is not limited herein.

In a possible implementation of the first aspect, the method further includes: obtaining real-time physiological data currently collected by a second electronic device; adjusting the first training plan to a second training plan when detecting that the real-time physiological data meets a first trigger condition for adjusting a training plan, where at least one exercise control parameter in the first training plan is different from that in the second training plan; and sending a second set of control instructions to the exercise apparatus based on the second training plan.

The second set of control instructions may be one or more control instructions sent by the first electronic device to the exercise apparatus for each action phase in the second training plan.

In a possible implementation of the first aspect, the real-time physiological data includes a real-time heart rate; and detecting that the real-time physiological data meets the first trigger condition for adjusting a training plan includes: detecting that the real-time heart rate is less than a lower limit of a target heart rate zone; or detecting that the real-time heart rate is greater than an upper limit of a target heart rate zone.

In a possible implementation of the first aspect, adjusting the first training plan to the second training plan includes:

when detecting that the real-time heart rate is less than the lower limit of the target heart rate zone, calculating, based on the lower limit, a load parameter increment corresponding to a next phase in the first training plan, and sending the load parameter increment to the exercise apparatus by using a second control instruction, where the second set of control instructions include the second control instruction; or

when detecting that the real-time heart rate is greater than the upper limit of the target heart rate zone, calculating, based on the upper limit, a load parameter decrement corresponding to a next phase in the first training plan, and sending the load parameter decrement to the exercise apparatus by using a third control instruction, where the second set of control instructions include the third control instruction.

In a possible implementation of the first aspect, before obtaining the real-time physiological data currently collected by the second electronic device, the method further includes: obtaining a current operating parameter of the exercise apparatus;

adjusting the first training plan to a third training plan based on a result of comparison between the current operating parameter and a target load parameter in a next phase in the first training plan, where at least one exercise control parameter in the first training plan is different from that in the third training plan; and

sending a third set of control instructions to the exercise apparatus based on the third training plan.

In a possible implementation of the first aspect, the exercise apparatus is a speed-type apparatus, the current operating parameter is a current speed, the target load parameter is a target speed, and the result of comparison between the current operating parameter and the target load parameter in the next phase in the first training plan includes: The current speed is less than the target speed; or the current speed is greater than the target speed.

In a possible implementation of the first aspect, when the current speed is less than the target speed, adjusting the first training plan to the third training plan includes any one of the following:

when detecting that a difference between the target speed and the current speed is less than a first difference threshold, sending the target speed to the exercise apparatus by using a fourth control instruction, to control a pace of the exercise apparatus to increase from the current speed to the target speed, where the third set of control instructions include the fourth control instruction;

when detecting that a difference between the target speed and the current speed is greater than or equal to a first difference threshold and is less than a second difference threshold, sending a difference between the target speed and a preset constant to the exercise apparatus by using a fifth control instruction, to control a pace of the exercise apparatus to increase from the current speed to the difference between the target speed and the preset constant, where the second difference threshold is greater than the first difference threshold, and the third set of control instructions include the fifth control instruction; or

when detecting that a difference between the target speed and the current speed is greater than a second difference threshold, obtaining the real-time physiological data currently collected by the second electronic device, and adjusting the first training plan based on the real-time physiological data.

For example, the first difference threshold may be a lower limit m of a preset speed difference interval [m, n], and the second difference threshold may be an upper limit n of the preset speed difference interval [m, n]. The preset constant may be, for example, a difference constant a. The fourth control instruction and the fifth control instruction may be, for example, acceleration control instructions for the treadmill.

In a possible implementation of the first aspect, when the current speed is greater than the target speed, adjusting the first training plan to the third training plan includes any one of the following:

when detecting that a difference between the current speed and the target speed is less than or equal to a third difference threshold, sending the target speed to the exercise apparatus by using a sixth control instruction, to control a pace of the exercise apparatus to decrease from the current speed to the target speed, where the third set of control instructions include the sixth control instruction; or

when detecting that a difference between the current speed and the target speed is greater than a third difference threshold, obtaining the real-time physiological data currently collected by the second electronic device, and adjusting the first training plan based on the real-time physiological data.

For example, the third difference threshold may correspond to a preset third threshold k of a speed difference. The sixth control instruction may be, for example, a speed reduction control instruction or a deceleration control instruction for the treadmill.

In a possible implementation of the first aspect, the exercise apparatus is a resistance-type apparatus, the current operating parameter is a current power, the target load parameter is a target power, and the result of comparison between the current operating parameter and the target load parameter in the next phase in the first training plan includes: The current power is not equal to the target power.

In a possible implementation of the first aspect, when the current power is not equal to the target power, adjusting the first training plan to the third training plan includes any one of the following:

when detecting that an absolute value of a difference between the current power and the target power is less than a fourth difference threshold, sending, to the exercise apparatus, a seventh control instruction instructing to maintain the current power for first preset duration;

when detecting that an absolute value of a difference between the current power and the target power is greater than or equal to a fourth difference threshold and is less than a fifth difference threshold, sending, to the exercise apparatus, an eighth control instruction instructing to maintain the current power for second preset duration, where the fifth difference threshold is greater than the fourth difference threshold; or

when detecting that an absolute value of a difference between the current power and the target power is greater than a fifth difference threshold, sending, to the exercise apparatus, an eighth control instruction instructing to maintain the current power for third preset duration, or obtaining the real-time physiological data currently collected by the second electronic device and adjusting the first training plan based on the real-time physiological data, where

the first preset duration is less than the second preset duration, and the second preset duration is less than the third preset duration.

For example, the fourth difference threshold may be a lower limit i of a preset power difference interval [i, j], and the fourth difference threshold may be an upper limit j of the preset power difference interval [i, j].

According to a second aspect, this application provides an exercise control method, applied to a first electronic device. The method includes:

detecting an exercise target parameter input operation of a user for an exercise apparatus;

controlling, in response to the exercise target parameter input operation, the exercise apparatus to execute a corresponding first training plan; and

when detecting that an exercise status of the user meets a preset condition, controlling the exercise apparatus to execute a corresponding second training plan, where at least one operating parameter of the exercise apparatus in the corresponding first training plan is different from that in the corresponding second training plan.

According to a third aspect, this application provides an electronic device, including one or more processors and one or more memories. The one or more memories store one or more programs. When the one or more programs are executed by the one or more processors, the electronic device is enabled to perform the exercise control method according to any one of the first aspect, the second aspect, and the possible implementations of the first aspect.

According to a fourth aspect, this application provides a computer-readable medium. The readable medium stores instructions. When the instructions are executed on a computer, the computer is enabled to perform the exercise control method according to any one of the first aspect, the second aspect, and the possible implementations of the first aspect.

BRIEF DESCRIPTION OF DRAWINGS

FIG. 1A to FIG. 1C are a diagram of an application scenario according to an embodiment of this application;

FIG. 2 is a diagram of a current exercise interface for adjusting a load of an exercise apparatus based on a heart rate;

FIG. 3 is a diagram of an implementation principle of an exercise control method according to an embodiment of this application;

FIG. 4 is a diagram of a structure of an electronic device according to an embodiment of this application;

FIG. 5 is a diagram of a hardware structure of an exercise apparatus according to an embodiment of this application;

FIG. 6A and FIG. 6B are a diagram of an implementation procedure of an exercise control method according to Embodiment 1 of this application;

FIG. 7a is a diagram of a health interface displayed when a health app is started according to Embodiment 1 of this application;

FIG. 7b is a diagram of an exercise control interface according to Embodiment 1 of this application;

FIG. 8a is a diagram of a training course list interface according to Embodiment 1 of this application;

FIG. 8b is a diagram of a training plan interface according to Embodiment 1 of this application;

FIG. 9 is a diagram of an implementation procedure of a running capability model for training a user according to Embodiment 1 of this application;

FIG. 10 is a diagram of an implementation procedure of adjusting a load of a treadmill based on collected real-time exercise data according to Embodiment 1 of this application;

FIG. 11 is a diagram of an implementation procedure of adjusting a load of a treadmill based on a real-time heart rate according to Embodiment 1 of this application;

FIG. 12 is a diagram of an exercise interface correspondingly displayed in an exercise process of a user according to Embodiment 1 of this application;

FIG. 13A to FIG. 13C are a diagram of a cycling exercise scenario according to Embodiment 2 of this application;

FIG. 14A and FIG. 14B are a diagram of an implementation procedure of another exercise control method according to Embodiment 2 of this application;

FIG. 15 is a diagram of an implementation procedure of adjusting a load of a spin bike based on collected real-time exercise data according to Embodiment 2 of this application;

FIG. 16 is a diagram of an implementation procedure of adjusting a load of a spin bike based on a real-time heart rate according to Embodiment 2 of this application; and

FIG. 17 is a block diagram of a structure of a software system of an electronic device according to an embodiment of this application.

DESCRIPTION OF EMBODIMENTS

To make objectives, technical solutions, and advantages of embodiments of this application clearer, the following describes the technical solutions in embodiments of this application in detail with reference to accompanying drawings and specific implementations of the specification.

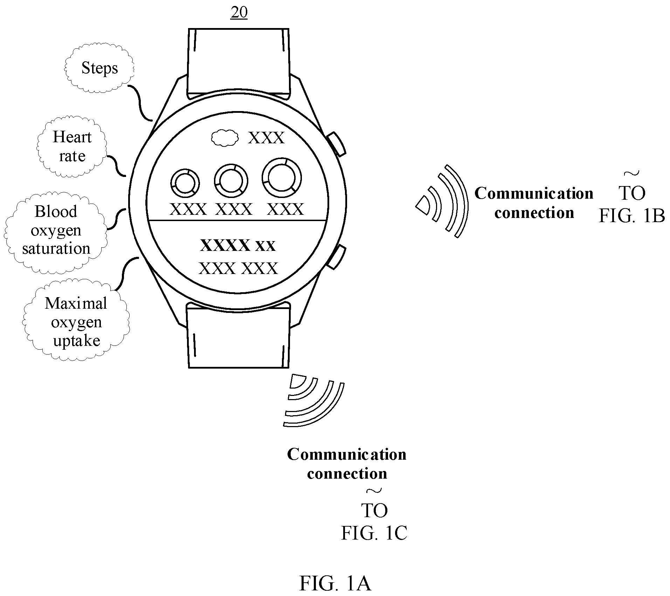

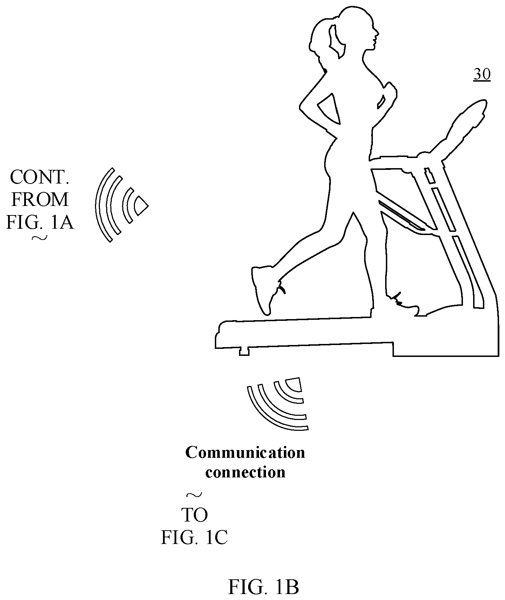

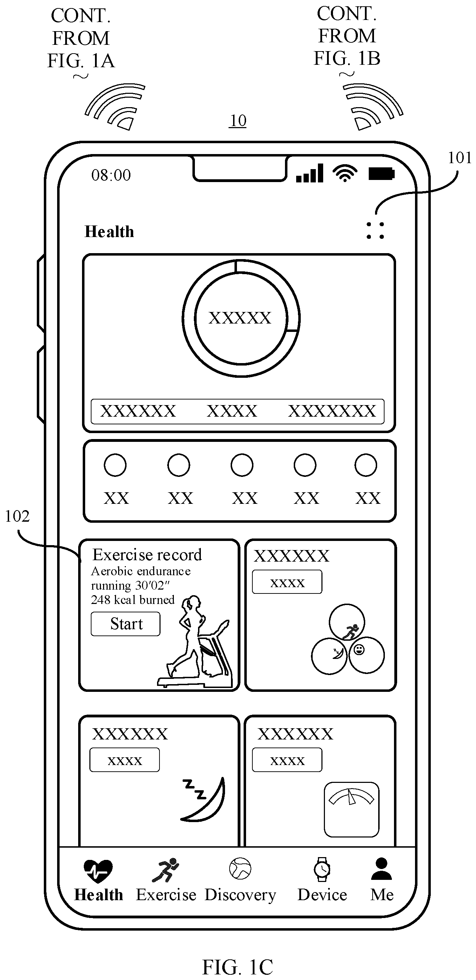

FIG. 1A to FIG. 1C are a diagram of an application scenario according to an embodiment of this application.

As shown in FIG. 1A to FIG. 1C, the scenario includes a mobile phone 10, a watch 20, and a treadmill 30 that establish communication connections to each other. The mobile phone 10 may run an application (application, APP) such as health, and a correspondingly displayed health interface 101 may display card information such as an exercise record 102 of a user. In some other embodiments, the mobile phone 10 may alternatively run another app having an exercise recording function. The scenario shown in FIG. 1A to FIG. 1C may also include another wearable device different from the watch 20, for example, a band or a headset. The treadmill 30 is a speed-type apparatus. In some other embodiments, the exercise apparatus in the scenario shown in FIG. 1A to FIG. 1C may alternatively be another speed-type exercise apparatus, for example, a climbing machine. This is not limited herein.

As shown in FIG. 1A to FIG. 1C, the exercise record 102 may display a record related to latest exercise performed by the user, for example, the user performs "Aerobic endurance running", "Time 30'02''", and "248 kcal burned". The "Aerobic endurance running" may be a training course selected by the user, and the course may be preset in the health app. In some other embodiments, the user may alternatively select another training course that matches a training objective to take exercise, for example, "Fat-burn run" or "Story run".

Still as shown in FIG. 1A to FIG. 1C, the watch 20 that is communicatively connected to the mobile phone 10 may collect exercise data such as real-time physiological data and steps of the user in an exercise process. The physiological data may include but is not limited to data such as a heart rate, a maximal oxygen uptake (maximal oxygen uptake, Vo2Max), and blood oxygen saturation. The exercise data such as the physiological data and the steps collected by the watch 20 may be synchronized to the mobile phone 10, to update display content on a related interface provided by the health interface 101 or the health app.

The treadmill 30 that is communicatively connected to the mobile phone 10 may collect apparatus parameters such as a speed (speed), an incline (incline), a distance target, and a time (time) target that are set by the user, and send the apparatus parameters to the mobile phone 10. Therefore, in a process in which the user takes exercise on the treadmill 30, the mobile phone 10 may directly control, based on whether heart rate data and the like collected by the watch 20 are within a preset range, for example, when the mobile phone 10 detects that a current heart rate of the user is lower than a preset normal heart rate zone, to increase a load of the treadmill 30, for example, increase a pace or an incline of the treadmill 30, without considering whether the user can bear an increased load based on an actual exercise capability.

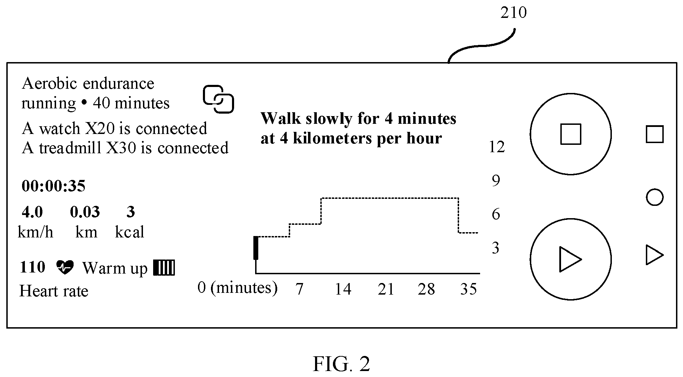

FIG. 2 is a diagram of a current exercise interface for adjusting a load of an exercise apparatus based on a heart rate.

As shown in FIG. 2, an exercise interface 210 may display a training course "Aerobic endurance running" selected by a user, and a time target is 40 minutes. In addition, the exercise interface 210 may display a currently connected device, for example, display "A watch X20 is connected" and "A treadmill X30 is connected".

In a warm up phase of the user at the beginning of exercise, an initial speed of a treadmill 30 may be low, for example, "4.0 km/h". In this case, a heart rate of the user increases as exercise time increases. For example, after the user walks slowly for 4 minutes at a speed of 4.0 km/h, the heart rate may increase from 95 to 110. However, the heart rate may be lower than a target heart rate zone expected to be reached in the warm up phase of the "Aerobic endurance running". In this case, a mobile phone 10 that displays the exercise interface 210 shown in FIG. 2 may directly send a speed adjustment instruction or an incline adjustment instruction to the treadmill 30, to control the treadmill 30 to increase a load. For example, the mobile phone 10 may control a pace of the treadmill 30 to directly increase from 4 km/h to 5 km/h, or the mobile phone 10 may control an incline of the treadmill 30 to directly increase from 5% to 10%. In this case, a load increment of the treadmill 30 may be large, which is not conducive to a physical function of the user to adapt to such adjustment. In addition, the adjusted speed and/or incline of the treadmill may not match an actual exercise capability of the user, causing some exercise safety problems.

In addition, if the heart rate of the user cannot reach a lower limit of the target heart rate zone in the warm up phase, the mobile phone 10 may increase a current heart rate of the user by controlling the treadmill 30 to continuously increase a load. For example, the mobile phone 10 may continuously send a speed adjustment instruction to the treadmill 30 to increase the pace, or send an incline adjustment instruction to increase the incline. This manner of frequently adjusting a speed, an incline, and the like of the treadmill also causes poor exercise experience of the user.

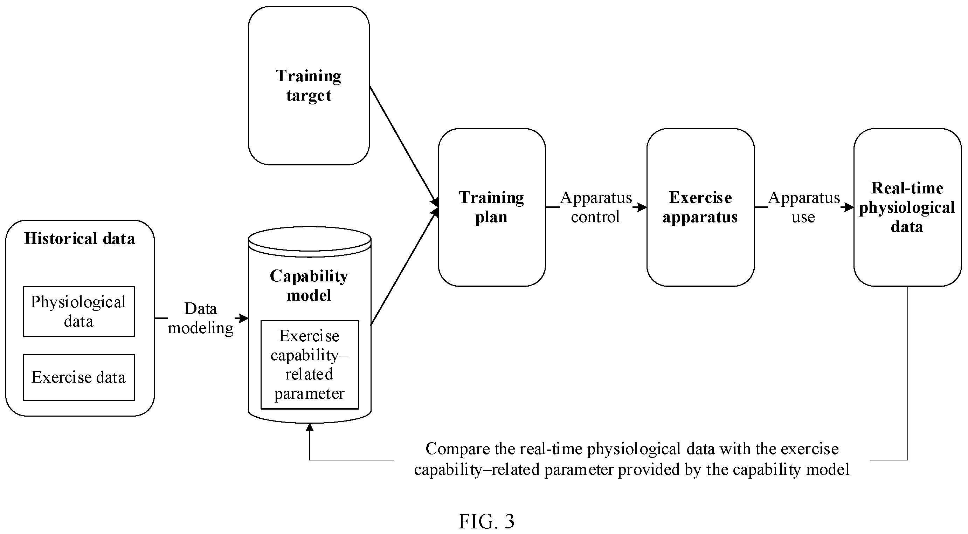

To resolve the foregoing problem, an embodiment of this application provides an exercise control method, applied to an electronic device. Specifically, as shown in FIG. 3, when detecting an exercise demand of a user, the electronic device may generate, based on a **training target** set by the user, for example, a distance target, a time target, an endurance enhancement target, and a cardiopulmonary improvement target that are set by the user, and with reference to an exercise capability-related parameter determined based on a historical exercise record of the user, for example, a load bearing range, a maximal heart rate, and a heart rate change zone that are of the user for a corresponding exercise apparatus, a **training plan** that matches an exercise capability of the user. The training plan may include exercise control parameters in a plurality of phases. An exercise control parameter in each phase may include, for example, but is not limited to, a target distance, target duration, a target heart rate, a target heart rate zone, a pace, a speed change range, a matched incline, and a matched resistance that needs to be achieved in each phase. In this way, a training plan suitable for the user can be generated based on a training target input by the user and an actual exercise capability of the user, to help achieve a better exercise effect and improve exercise safety of the user.

For example, a running capability of a user A is strong, and a running capability of a user B is weak. When training targets input by the user A and the user B are the same, there may be a high treadmill pace in a fast walking phase in a training plan generated for the user A, and there may be a low treadmill pace in a slow walking phase in a training plan generated for the user B.

Further, still as shown in FIG. 3, in a process in which the user takes exercise based on the generated training plan, the electronic device may further dynamically adjust, based on the collected **real-time physiological data,** for example, a heart rate of the user, a maximal oxygen uptake during current exercise, and oxygen saturation, training content in a next phase that is to be started in the generated training plan, including adjusting target duration of the next phase, and a pace, an incline, or a resistance of a corresponding exercise apparatus. In addition, in a process in which the user takes exercise, when detecting that a difference between a heart rate of the user collected in real time and a target heart rate that matches the phase is large, or a heart rate that deviates from a target heart rate zone is large, the electronic device may also trigger timely adjustment of an exercise control parameter configured in a current phase.

It may be understood that, the generated training plan can be used to control configuration parameters such as a load and time of a corresponding exercise apparatus. In a process in which the user uses the exercise apparatus, real-time physiological data collected by the user is compared with an exercise capability-related parameter of the user, so that the training plan may be further optimized and adjusted. For example, in a training plan initially generated based on an exercise capability of the user, a pace in a fast walking phase is 5 km/h. However, in a process in which the user takes exercise based on the training plan, it is detected that a real-time heart rate of the user in a slow walking phase before the fast walking phase is already high, indicating that a current physiological status of the user may be weaker than that in normal cases. In this case, a pace in the to-be-started fast walking phase in the training plan may be adjusted to 4.5 km/h. In this way, the exercise control solution provided in this application can adaptively adjust, based on a change of the exercise capability of the user, the training plan that matches the exercise capability of the user, and can dynamically adjust the training plan based on an actual physiological status of the user in an exercise process of the user. This can control exercise safety based on the actual physiological status of the user in the exercise process, and help achieve a better exercise effect.

In addition, according to the exercise control method provided in this application, a corresponding threshold parameter and a segmental adjustment policy are further preset for an adjustment amplitude and an adjustment frequency of training content in each phase in the training plan, to avoid poor user experience caused by excessively frequent adjustment or an excessively large adjustment amplitude.

Still as shown in FIG. 3, the exercise capability-related parameter may be provided by a trained capability model, and the capability model may be obtained through training based on historical data such as physiological data and exercise data in the historical exercise record of the user.

Each phase in the generated training plan may also be marked with different training types for differentiation. For example, different phases may be marked as training types such as "slow walking", "fast walking", and "fast running" based on a corresponding pace and a speed change range. In some other embodiments, each phase may alternatively be marked as a training type such as "low resistance", "medium resistance", or "high resistance" based on a resistance parameter of a matched exercise apparatus. This is not limited herein.

It may be understood that an electronic device to which the exercise control method provided in this embodiment of this application is applicable may include but is not limited to a mobile phone, a tablet computer, a desktop computer, a laptop computer, a handheld computer, a netbook, an augmented reality (augmented reality, AR) device/a virtual reality (virtual reality, VR) device, a smart television, a wearable device such as a smart watch, a server, a mobile email device, a head unit, a portable game console, a portable music player, a reader device, a television set embedded with or coupled to one or more processors, or another electronic device that can access a network.

For example, the wearable device in this embodiment of this application may be a wired headset, a wireless headset (for example, a TWS Bluetooth headset, a neck-mounted Bluetooth headset, or a head-mounted Bluetooth headset), a smartwatch, a smart wristband, smart glasses, a smart ankle band, a smart ring, a smart necklace, or the like. A specific form of the wearable device is not specifically limited in this application.

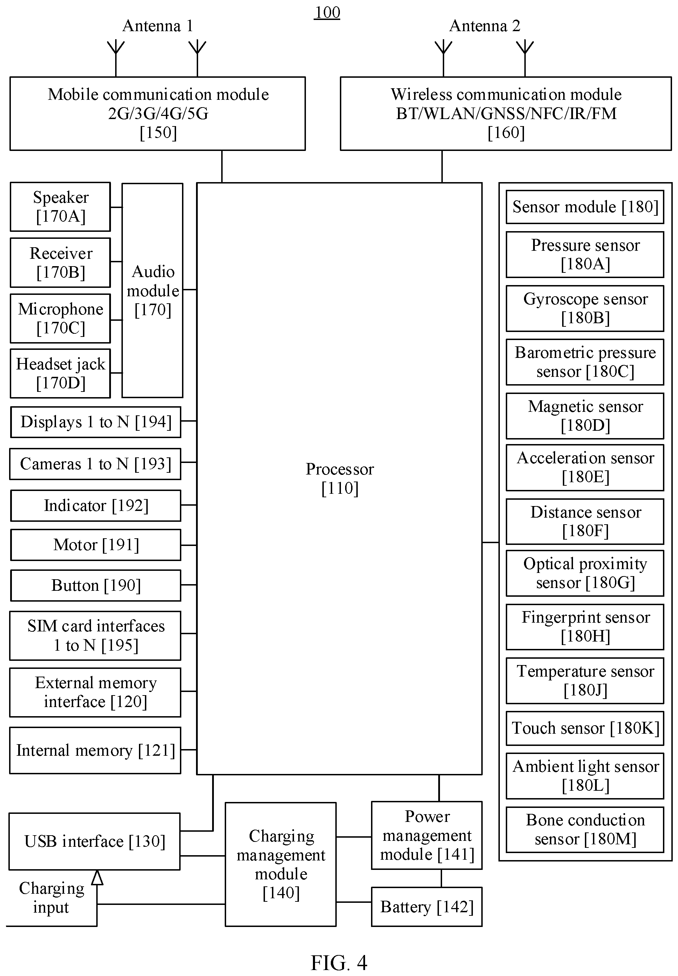

FIG. 4 is a diagram of a structure of an electronic device 100 according to an embodiment of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, or a combination of some components, or splits from some components, or a different component layout. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on instruction operation code and a time sequence signal, to complete control of instruction reading and instruction execution.

In this embodiment of this application, the processor 110 may generate an operation control signal by using the controller, to complete corresponding steps performed by an electronic device such as the mobile phone 10, the watch 20, or the treadmill 30 in the scenario shown in FIG. 1A to FIG. 1C to implement the exercise control method provided in this application. For a specific implementation process, refer to detailed descriptions in Embodiment 1 and Embodiment 2 below. Details are not described herein again.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to exchange data between the electronic device 100 and a peripheral device, or may be configured to connect to a headset for playing an audio through the headset. The interface may be further configured to connect to another electronic device such as an AR device.

It may be understood that an interface connection relationship between the modules that is illustrated in this embodiment of the present invention is merely an example for description, and does not constitute a limitation on a structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from the charger. The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to: transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes a 2G/3G/4G/5G or the like.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-CDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 implements a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel.

The electronic device 100 may implement a photographing function through the ISP, the camera 193, the video codec, the GPU, the display 194, the application processor, and the like. The ISP may be configured to process data fed back by the camera 193. The camera 193 is configured to capture a static image or a video. An optical image of an object is generated through a lens, and is projected onto a photosensitive element. The digital signal processor is configured to process a digital signal, and may process another digital signal in addition to the digital image signal. The video codec is configured to compress or decompress a digital video. The electronic device 100 may support one or more video codecs. Therefore, the electronic device 100 may play or record videos in a plurality of coding formats, for example, moving picture experts group (moving picture experts group, MPEG)-1, MPEG-2, MPEG-3, and MPEG-4.

The external memory interface 120 may be configured to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a sound playing function or an image playing function), and the like. The data storage area may store data (such as audio data and an address book) and the like that are created during use of the electronic device 100. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The electronic device 100 may implement an audio function, for example, music playing and recording, through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to: encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal.

The receiver 170B, also referred to as an "earpiece", is configured to convert an electrical audio signal into a sound signal.

The microphone 170C, also referred to as a "mike" or a "mic", is configured to convert a sound signal into an electrical signal.

The headset jack 170D is configured to connect to a wired headset. The headset jack 170D may be the USB interface 130, or may be a 3.5 mm open mobile terminal platform (open mobile terminal platform, OMTP) standard interface, or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on a change in the capacitance. When a touch operation is performed on the display 194, the electronic device 100 detects intensity of the touch operation by using the pressure sensor 180A. The electronic device 100 may also calculate a touch location based on a detection signal of the pressure sensor 180A. In some embodiments, touch operations that are performed in a same touch position but have different touch operation intensity may correspond to different operation instructions.

The gyroscope sensor 180B may be configured to determine a moving posture of the electronic device 100. In some embodiments, an angular velocity of the electronic device 100 around three axes (namely, axes x, y, and z) may be determined through the gyroscope sensor 180B. In this embodiment of this application, based on judgment of a moving posture of the electronic device 100, some preset algorithm models may be combined to identify a moving posture of the user, for example, identify that the user is running or cycling.

The acceleration sensor 180E may detect accelerations of the electronic device 100 in various directions (usually on three axes). When the electronic device 100 is still, a magnitude and a direction of gravity may be detected. The acceleration sensor 180E may be further configured to identify a posture of the electronic device, and is used in an application such as switching between a landscape mode and a portrait mode or a pedometer.

The touch sensor 180K is also referred to as a "touch component". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor to determine a type of a touch event. A visual output related to the touch operation may be provided on the display 194. In some other embodiments, the touch sensor 180K may alternatively be disposed on a surface of the electronic device 100 at a location different from that of the display 194. In this embodiment of this application, for example, when the electronic device 100 is the mobile phone 10, the mobile phone 10 may receive, by using the touch sensor 180K, an input operation performed by the user on a related interface of a health app. For another example, when the electronic device 100 is the watch 20, the watch 20 may also receive, by using the touch sensor 180K, related operations performed by the user on an exercise function interface such as starting to exercise or end exercise, as well as related operations on a function interface such as heart rate detection.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100.

It may be understood that, in this embodiment of this application, the electronic device 100 may be, for example, the electronic device such as the mobile phone 10 or the watch in the scenario shown in FIG. 1A to FIG. 1C.

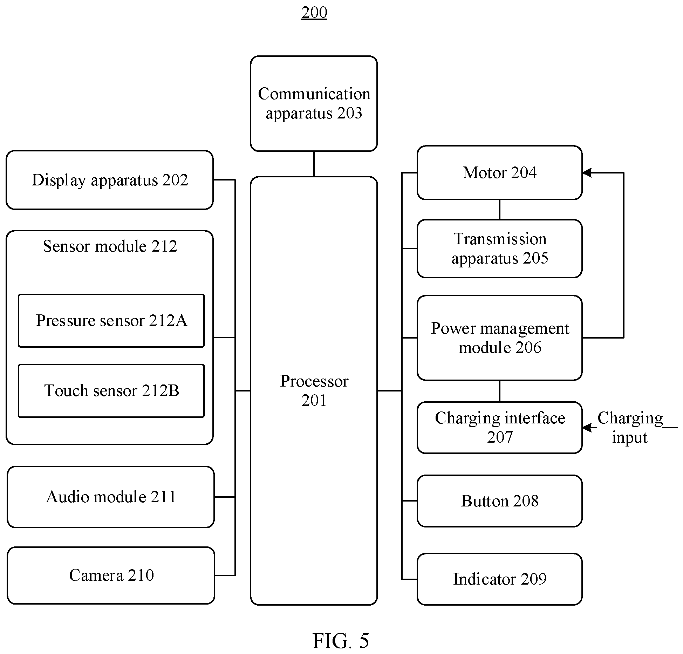

FIG. 5 is a diagram of a hardware structure of an exercise apparatus according to an embodiment of this application.

It may be understood that the exercise apparatus may be an intelligent exercise device. A specific type of the exercise apparatus is not limited in embodiments of this application. The exercise apparatus may be the treadmill 30, an elliptical trainer, a spin bike, a rowing machine, a climbing machine, or the like. It may be understood that, when the exercise apparatus corresponds to different types of exercise devices, some hardware structures may be added or reduced.

As shown in FIG. 5, the exercise apparatus 200 may include a processor 201, a display apparatus 202, a communication apparatus 203, a motor 204, a transmission apparatus 205, a power management module 206, a charging interface 207, a button 208, an indicator 209, a camera 210, an audio module 211, and a sensor module 212. The audio module 211 may include a speaker 211A, a microphone 211B, an audio interface 211C. The sensor module 212 may include a pressure sensor 212A, a touch sensor 212B, and the like.

For related processing functions of the processor 201, refer to related function descriptions of the processor 110. For related display functions of the display apparatus 202, refer to related function descriptions of the display 194. For related communication functions of the communication apparatus 203, refer to related function descriptions of the wireless communication module 160. For related functions of the power management module 206 and the charging interface 207, refer to related function descriptions of the charging management module 140. For related functions of the button 208 and the indicator 209, refer to related function descriptions of the button 190 and the indicator 192 respectively. For related audio functions of the audio module 211, refer to related function descriptions of the audio module 170. For related functions of the sensor module 212, refer to related function descriptions of the sensor module 180. For the pressure sensor 212A and the touch sensor 212B included in the sensor module 212, refer to related function descriptions of the pressure sensor 180A and the touch sensor 180K included in the sensor module 180. Details are not described herein again.

The motor 204 may convert electric power into mechanical power, to provide mechanical power for the transmission apparatus 205. The motor may include a direct current motor and an alternating current motor. The motor is generally connected to the transmission apparatus 205 and is configured to drive the transmission apparatus 205.

The transmission apparatus 205 is configured to directly assist a user in exercising. For example, in a treadmill, the transmission apparatus 205 may include a roller and a running belt. The roller may transmit power of a motor to the running belt, and the running belt continuously transmits the power, so that the user can run on the running belt. The user can adjust a transmission speed of the running belt to adjust a running speed.

Based on the scenario shown in FIG. 1A to FIG. 1C and the structure shown in FIG. 4 and FIG. 5, the following describes in detail, with reference to specific embodiments, implementation processes of exercise control solutions provided in embodiments of this application in different exercise scenarios.

With reference to Embodiment 1, the following first describes a specific implementation process of an exercise control solution provided in this application in a running exercise scenario.

**Embodiment 1**

A scenario described in this embodiment of this application is the running exercise scenario shown in FIG. 1A to FIG. 1C. It may be understood that, as shown in FIG. 1A to FIG. 1C, the running exercise scenario may include a mobile phone 10, a watch 20, and a treadmill 30.

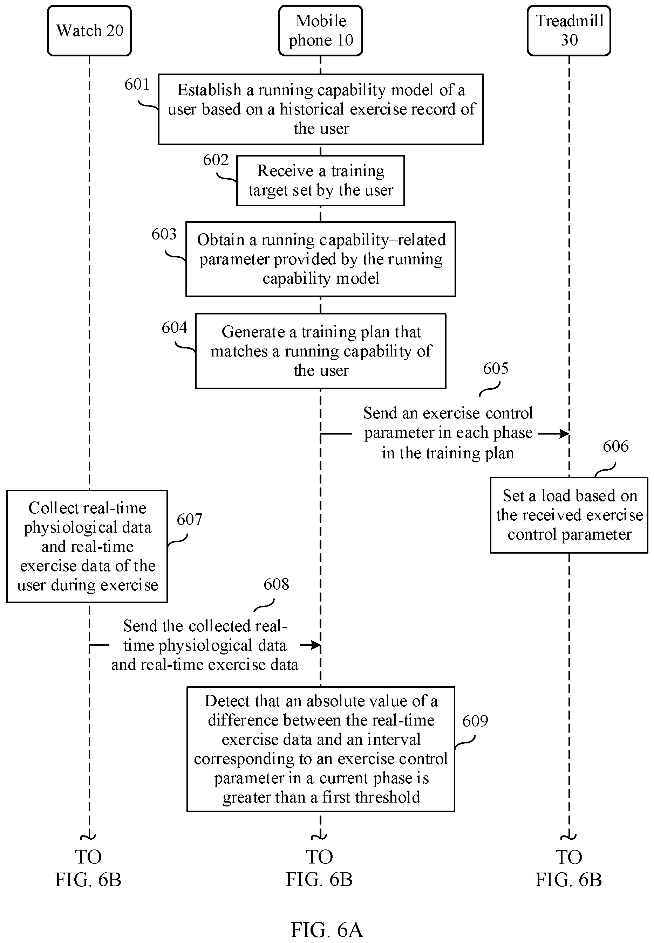

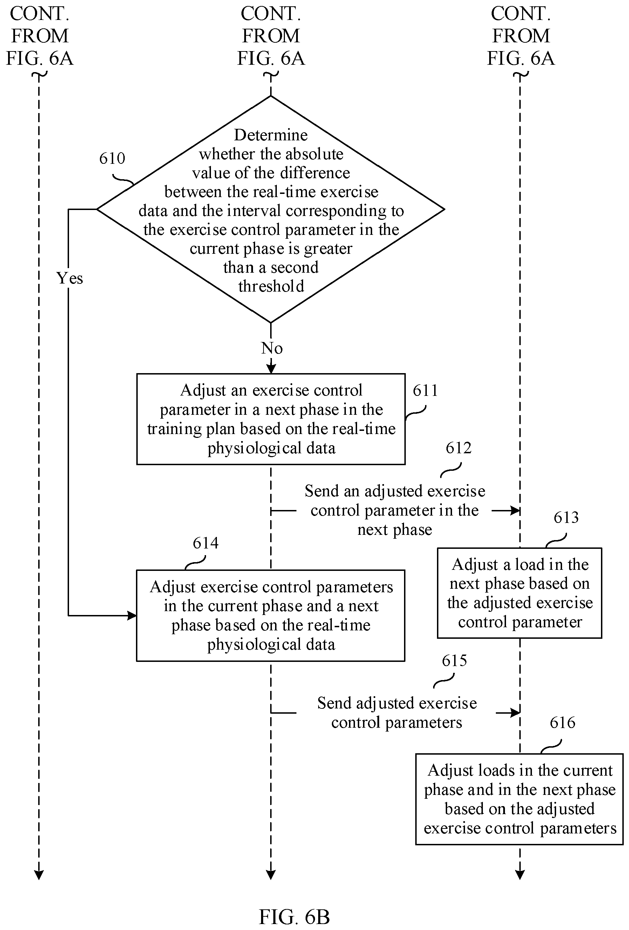

Based on the scenario shown in FIG. 1A to FIG. 1C, FIG. 6A and FIG. 6B are a diagram of an implementation procedure of an exercise control method according to an embodiment of this application. The implementation procedure is an interaction procedure implemented by the mobile phone 10, the watch 20, and the treadmill 30 that perform each step.

It should be further stated that, in embodiments of this application, the steps in the method and the procedure are numbered for ease of reference, not to limit a sequence. If there is a sequence between the steps, text descriptions shall prevail.

As shown in FIG. 6A and FIG. 6B, specifically, the procedure includes the following steps.

601: The mobile phone 10 establishes a running capability model of a user based on a historical exercise record of the user.

For example, the mobile phone 10 may first establish an exercise capability model of the user based on the historical exercise record of the user. The exercise capability model is referred to as a capability model for short below. The capability model may describe an actual exercise capability of the user to some extent. In this embodiment of this application, the capability model may be the running capability model of the user. The historical exercise record of the user may include exercise data recorded in different types of exercise that the user participates in and physiological data during corresponding exercise. In this embodiment of this application, the different types of exercise may be, for example, exercise performed by the user on the treadmill 30, such as running, fast walking, inclined running, or inclined walking. In some other embodiments, the different types of exercise may alternatively be cycling on a spin bike, exercise on an elliptical trainer, or exercise performed by the user by using an exercise apparatus such as a rowing machine or a strength training station. This is not limited herein.

It may be understood that the physiological data included in the historical exercise record of the user may include but is not limited to a gender, an age (age), a heart rate reserve (heart rate reserve, HRR), a maximal heart rate (maximal heart rate, MaxHR), a rest heart rate (rest heart rate, RestHR), a maximal oxygen uptake (Vo2Max), and the like of the user. The exercise data included in the historical exercise record may include but is not limited to a distance, exercise duration, data related to a heart rate curve, a heart rate fluctuation range, a speed in each time period, calories burned, an action frequency, a resistance change curve, and the like.

It may be understood that the capability model established based on the historical exercise record may include, for example, a maximum load value that is of the exercise apparatus and that the user can bear, a maximal heart rate reached by the user in an exercise process, a correspondence between a heart rate change zone of the user and training intensity, and a correspondence between a calorie burned value and the training intensity, that are obtained through statistics collection.

A specific process of establishing the running capability model of the user based on the historical exercise record of the user is described in detail below. Details are not described herein again.

602: The mobile phone 10 receives a training target set by the user.

For example, the user may set a training target of current exercise, select a training course that the user is interested in, or the like on an exercise control interface displayed on the mobile phone 10. The training course displayed by the mobile phone 10 may be a training plan with a corresponding training target, where the training plan is preset on an application such as a health app and that is selected by the user.

If the user selects an existing training course, the mobile phone 10 may obtain an exercise control parameter that is set by default in each phase in the corresponding training course. Further, the mobile phone 10 may continue to perform step 603 and step 604, to generate a training plan that matches an exercise capability of the user.

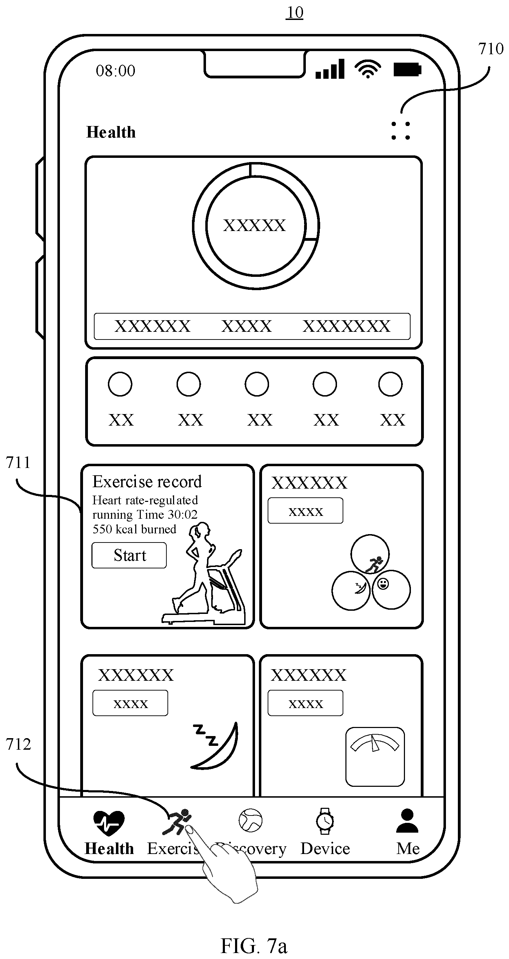

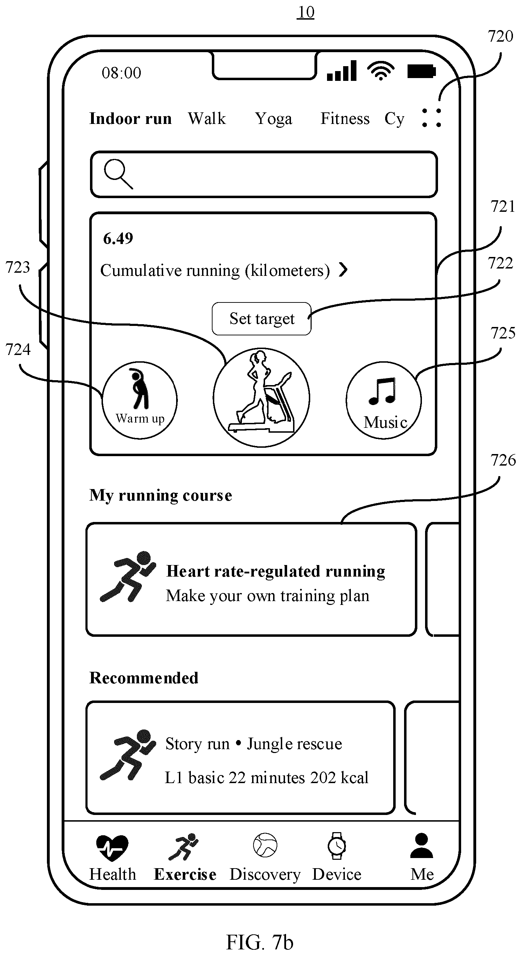

For example, FIG. 7a and FIG. 7b each are a diagram of a process of opening an exercise control interface to set a training target.

FIG. 7a is a diagram of a health interface displayed when a health app is started.

As shown in FIG. 7a, after running the health app in response to a user operation, the mobile phone 10 may display a health interface 710. A latest exercise record may be displayed on an exercise record card 711 on the health interface 710. For example, the displayed and recorded training course is "Heart rate-regulated running", and "Time 30:02" that is recorded indicates that exercise duration is 30'02". The exercise record may further include calories burned by the user for completing a corresponding exercise course, for example, "550 kcal burned" shown in FIG. 7a.

An interface switching control, for example, an Exercise control 712 shown in FIG. 7a, is further displayed in a lower part of the health interface 710. When detecting that the user taps the Exercise control 712, the mobile phone 10 may display an exercise control interface 720 shown in FIG. 7b.

As shown in FIG. 7b, the user may set a training target in a training target input box 721 displayed on the exercise control interface 720. For example, the user may tap a setting control 722 to set a distance target, a time target, a calorie burning target, and the like. The distance target may be set to, for example, 1 km, 3 km, 5 km, or 10 km; the time target may be set to, for example, 10 minutes, 20 minutes, or 30 minutes; and the calorie burning target may be set to, for example, 100 kcal or 200 kcal, which are not enumerated here. In some other embodiments, the training target set by the user may alternatively be some functional training targets, for example, endurance enhancement training and cardiopulmonary improvement training. Such training targets may be reflected by setting the distance target or the time target.

After the target is set, the user may tap a start control 723. The mobile phone 10 may continue to perform, based on the training target set by the user, step 603 and step 604 to generate a training course that corresponds to the set target. Before the training starts, the user may also tap a Warm up control 724 to enter a preset warm-up course to perform warm-up training, or may tap a Music control 725 to set a piece of music or the like that the user wants to listen to during exercise.

Still as shown in FIG. 7b, the user may select a training course displayed on the exercise control interface 720 for training, for example, a "Heart rate-regulated running" course 726 shown in FIG. 7b. In this case, the mobile phone 10 may obtain, as an initial parameter, an exercise control parameter in each phase in one or more preset training plans corresponding to the course 726, and complete adjustment of the initial parameter after performing step 603 and step 604. The one or more preset training plans corresponding to the "Heart rate-regulated running" course 726 are described in the following corresponding steps with reference to the accompanying drawings. Details are not described herein again.

It may be understood that, in some other embodiments, an interface on which the mobile phone 10 receives the training target set by the user may be different from other interfaces shown in FIG. 7a and FIG. 7b, and a manner in which the mobile phone 10 receives the training target set by the user may alternatively be another manner, for example, receives the training target input by the user by using the watch 20. This is not limited herein in this application.

603: The mobile phone 10 obtains a running capability-related parameter provided by the running capability model.

For example, when detecting an exercise demand of the user, the mobile phone 10 may obtain a running capability-related parameter of a corresponding user based on the trained or updated running capability model. It may be understood that the running capability-related parameter may be a parameter updated based on a recent exercise record of the user, or may be a capability model training parameter obtained through statistics collection/training based on all historical exercise records of the user. This is not limited herein. The exercise demand of the user may be detected when the mobile phone 10 performs step 603 of receiving the training target set by the user, or when the mobile phone 10 detects that the user starts the health app. This is not limited herein.

In some other embodiments, the mobile phone 10 may first perform step 603 of obtaining the running capability-related parameter of the user, and then perform step 602 of receiving the training target set by the user. This is not limited herein.

604: The mobile phone 10 generates a training plan that matches a running capability of the user.

For example, the mobile phone 10 may generate, based on the training target set by the user and with reference to the running capability-related parameter provided by the running capability model of the user, the training plan that matches the running capability of the user. The training plan may include exercise control parameters in a plurality of phases.

For example, as shown in Table 1, the training plan may include a training target/training course, action phases 1 to N, an exercise control parameter corresponding to each phase, and the like.

Table 1

| Training target/Training course | Action phase | Exercise control parameter |
|---|---|---|
| 3 km target fast walking/heart rate- | Phase 1 | Training type: slow walking<br>Target distance: 1 km |
| regulated running | | Target heart rate and fluctuation range: 105 bpm\|[100, 110] bpm<br>Target speed and fluctuation range: 4 km/h\|[3.5, 4.5] km/h<br>Target incline: 5% |
| | Phase 2 | Training type: fast walking<br>Target distance: 1.5 km<br>Target heart rate and fluctuation range: 115 bpm\|[110, 120] bpm<br>Target speed and fluctuation range: 6 km/h\|[5, 6.5] km/h<br>Target incline: 5% |
| | ... | ... |
| | Phase N | Training type: fast walking<br>... |

In this embodiment of this application, a training control parameter in each phase may include a parameter such as a training type, a target distance, target duration, a target heart rate and a fluctuation range, a target speed and a fluctuation range, and a target incline in each phase. For values of the foregoing parameters, refer to specific examples in Table 1. The target heart rate fluctuation range may also be described as a target heart rate zone in some other embodiments, and the target speed fluctuation range may also be described as a speed change range in some other embodiments.

In the foregoing generated training plan, a manner of determining the exercise control parameter in each phase based on the training target set by the user and the running capability-related parameter of the user is described in detail below. Details are not described herein again.

It may be understood that the target speed, the fluctuation range, and the target incline may be used as related parameters for setting a load of the treadmill. The mobile phone 10 may send these parameters to the treadmill 30 when performing step 605, to set the load of the treadmill.

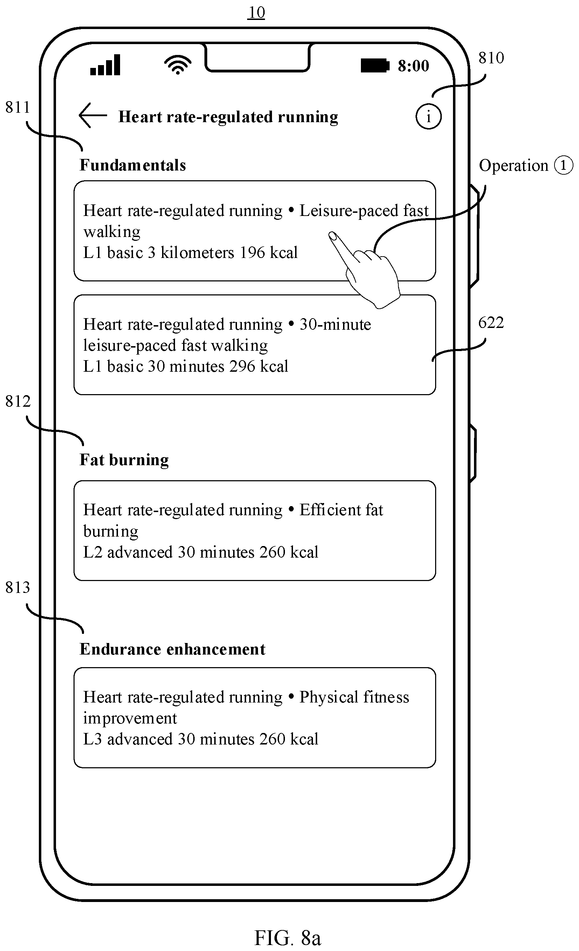

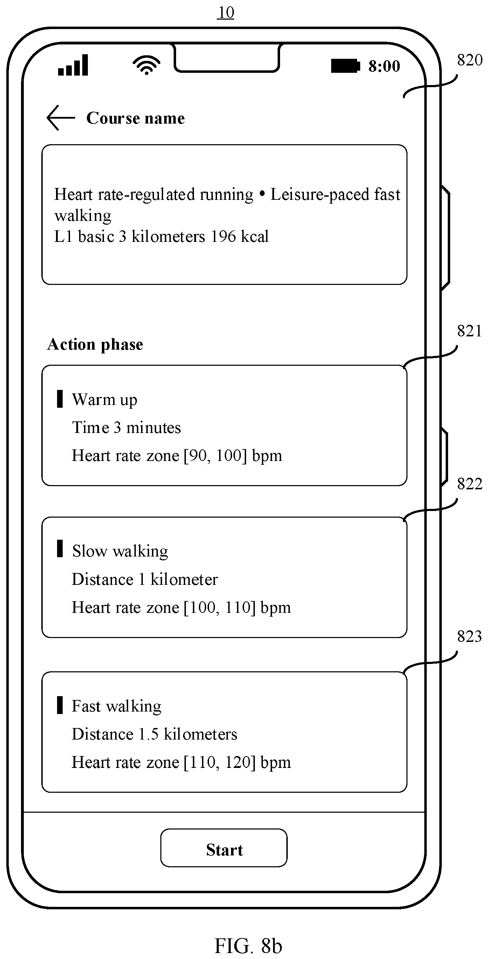

FIG. 8a and FIG. 8b are diagrams of interfaces corresponding to some training plans according to this embodiment of this application.

FIG. 8a shows a training course list interface 810. As shown in FIG. 8a, the course list interface 810 displays a plurality of courses related to "Heart rate-regulated running", including a "Fundamentals" course 811, a "Fat burning" course 812, an "Endurance enhancement" course 813, and the like. It may be understood that these courses may be named according to different training targets, and in some other embodiments, other naming manners may alternatively be used.

Refer to an "Operation ①" shown in FIG. 8a. For example, the user taps a "Heart rate-regulated running•Leisure-paced fast walking" course in the "Fundamentals" course 811, and the mobile phone 10 may further display a training plan interface 820 shown in FIG. 8b in response to the user operation.

As shown in FIG. 8b, the training plan interface 820 may display a plurality of action interfaces, for example, a warm up phase 821, a slow walking phase 822, and a fast walking phase 823. A display box corresponding to each phase may display some exercise control parameters in a corresponding phase. For example, a display box corresponding to the warm up phase 821 may display training time of "3 minutes" and a heart rate zone of "[90, 100] bpm" in the phase. A display box corresponding to the slow walking phase 822 may display a training distance of " 1 km" and a heart rate zone "[100, 110] bpm" in the phase. A display box corresponding to the fast walking phase 823 may display a training distance of " 1.5 km" and a heart rate zone "[110, 120] bpm" in the phase.

It may be understood that a plurality of action phases and an exercise control parameter in each phase in the training plan shown in FIG. 8b may match an actual exercise capability of the user. For different users, action phases included in corresponding training plans may be different, and exercise control parameters in each phase may also be different. In some other embodiments, an interface for displaying the training course and the training plan may alternatively be an interface style different from that in FIG. 8a and FIG. 8b. This is not limited herein.

605: The mobile phone 10 sends the exercise control parameter in each phase in the training plan to the treadmill 30.

For example, the mobile phone 10 may send, to the treadmill 30 based on the generated training plan, the exercise control parameters corresponding to the action phases included in the training plan. It may be understood that the exercise control parameters sent to the treadmill 30 may include a load-related control parameter that needs to be set on the treadmill 30, for example, a target speed and a change range, and a target incline, or may include target time or a target distance of a corresponding phase.

606: The treadmill 30 sets a load based on the received exercise control parameter.

For example, the treadmill 30 sets a corresponding load based on the received exercise control parameter. For example, the treadmill 30 may set a pace based on the received target speed and change range, and set an incline based on the received target incline.

It may be understood that, for different types of exercise apparatuses, loads set based on corresponding exercise control parameters are also different. For example, a parameter such as a cycling resistance may be set for a spin bike. For details, refer to related descriptions in the following Embodiment 2. Details are not described herein again.

607: The watch 20 collects real-time physiological data and real-time exercise data of the user during exercise.

For example, in a process in which the user takes exercise based on the training plan, the watch 20 or another wearable device worn by the user can collect the real-time physiological data of the user. The real-time physiological data may be, for example, a real-time heart rate. In some other embodiments, the real-time physiological data may alternatively be a parameter such as a maximal oxygen uptake during a corresponding exercise period. This is not limited herein.

It may be understood that the watch 20 may receive, based on a communication connection established between the watch 20 and the treadmill 30, a trigger signal indicating that the user starts to exercise on the treadmill 30, and automatically trigger collection of physiological data of the user. In some other embodiments, the watch 20 may alternatively trigger collection of the real-time physiological data of the user based on an operation such as tapping to start exercise by the user on a watch interface.

608: The watch 20 sends the collected real-time physiological data and real-time exercise data to the mobile phone 10.

For example, the real-time physiological data collected by the watch 20 may be sent to the mobile phone 10 in real time based on a communication connection established between the watch 20 and the mobile phone 10. Further, the mobile phone 10 may perform the following adjustment process in step 609 to step 616 based on the received real-time physiological data and real-time exercise data. For details, refer to related descriptions in the following corresponding steps. Details are not described herein again.

609: The mobile phone 10 determines that an absolute value of a difference between the real-time exercise data and an interval corresponding to an exercise control parameter in a current phase is greater than a first threshold.

For example, the real-time exercise data that is detected by the watch 20 and received by the mobile phone 10 may be greater than an upper limit of an interval corresponding to a corresponding exercise control parameter, or may be less than a lower limit of the interval corresponding to the corresponding exercise control parameter. For example, the real-time exercise data is a real-time speed of the user. In the phase 2 shown in Table 1, the real-time speed may be greater than an upper limit 2.5 km/h of the target speed interval [1.2, 2.5] km/h in Table 1, or may be less than a lower limit 1.2 km/h of the target speed interval. To measure a difference between the real-time speed and a target speed interval in a corresponding phase, the mobile phone 10 may calculate a difference between the real-time speed and the lower limit 1.2 km/h when the real-time speed is low, and take an absolute value, or may calculate a difference between the real-time speed and the upper limit 2.5 km/h when the real-time speed is high, and take an absolute value.

Further, the mobile phone 10 may compare the absolute value of the difference obtained through calculation with a corresponding preset difference threshold, to determine whether the real-time speed of the user has a large deviation. In step 609, the mobile phone 10 may compare the absolute value of the difference obtained through calculation with a smaller preset first threshold. If the absolute value of the difference is greater than the first threshold, the mobile phone 10 may further perform step 610, to determine whether a difference between the real-time speed and the target speed interval in the corresponding phase is large, so as to adaptively adjust the training plan.

610: The mobile phone 10 determines whether the absolute value of the difference between the real-time exercise data and the interval corresponding to the exercise control parameter in the current phase is greater than a second threshold, where the second threshold is greater than the first threshold.

If the absolute value is greater than the second threshold, it indicates that the difference between the real-time exercise data and the interval corresponding to the exercise control parameter in the current phase is large. In this case, the mobile phone 10 needs to perform an adjustment process of step 614 to step 616.

If the absolute value is not greater than the second threshold, it indicates that the difference between the real-time exercise data and the interval corresponding to the exercise control parameter in the current phase is small. In this case, the mobile phone 10 may adjust the training plan by performing an adjustment process of steps 611 to 613.

For example, the mobile phone 10 may compare the absolute value of the difference obtained through calculation in step 609 with the larger preset second threshold.

611: The mobile phone 10 adjusts an exercise control parameter in a next phase in the training plan based on the real-time physiological data.

For example, when performing step 609 and step 610 to determine that a difference between the real-time physiological data and the interval corresponding to the exercise control parameter in the current phase is small, the mobile phone 10 may adjust only the exercise control parameter in the next phase in the current training plan.

A specific process in which the mobile phone 10 adjusts the training plan based on the real-time physiological parameter is described in detail below. Details are not described herein again.

612: The mobile phone 10 sends an adjusted exercise control parameter in the next phase to the treadmill 30.

For example, after completing adjustment of the exercise control parameter in the next phase, the mobile phone 10 may send the adjusted exercise control parameter in the next phase to the treadmill 30.

In some other embodiments, the mobile phone 10 may alternatively send, to the treadmill 30, the training plan in which the exercise control parameter in the next phase is adjusted and updated. The updated training plan may include an exercise control parameter that is correspondingly updated in each phase. This is not limited herein.

613: The treadmill 30 adjusts a load in the next phase based on the adjusted exercise control parameter.

For example, the treadmill 30 may adjust the load in the next phase based on a load-related parameter in the received adjusted exercise control parameter in the next phase. A specific adjustment process is described in detail below. Details are not described herein again.

614: The mobile phone 10 adjusts exercise control parameters in the current phase and a next phase based on the real-time physiological data.

For example, when performing step 609 and step 610 to determine that a difference between the real-time physiological data and the interval corresponding to the exercise control parameter in the current phase is large, the mobile phone 10 may adjust exercise control parameters in a current action phase and the next phase in the current training plan.

A specific process in which the mobile phone 10 adjusts the training plan based on the real-time physiological parameter is described in detail below. Details are not described herein again.

615: The mobile phone 10 sends adjusted exercise control parameters to the treadmill 30.

For example, the treadmill 30 may adjust a corresponding load based on a load-related parameter in the received exercise control parameter. The exercise control parameter received by the treadmill 30 at this time includes the adjusted exercise control parameters in the current phase and the next phase. It may be understood that, in a current phase of exercise of the user, timely adjustment needs to be performed only when a physiological parameter such as a real-time heart rate of the user seriously deviates from a safe heart rate zone corresponding to the target heart rate zone, to ensure exercise safety of the user.

616: The treadmill 30 adjusts loads in the current phase and the next phase based on the adjusted exercise control parameters.

For example, the treadmill 30 may adjust the load in the current phase based on the adjusted load-related parameter in the current phase, and adjust the load in the next phase based on the adjusted load-related parameter in the next phase. A specific adjustment process is described in detail below. Details are not described herein again.

It may be understood that, in step 613 and step 616, a process in which the treadmill 30 adjusts a corresponding load based on the adjusted exercise control parameter in the corresponding phase may also be adjusted by using a preset segmental adjustment policy, and an adjustment frequency of the adjustment process may also be controlled based on a preset frequency threshold. This can avoid discomfort of the user due to great or frequent load adjustment in an exercise process of the user.

The following uses an example in which the user takes exercise on the treadmill 30, to specifically describe a detailed process of establishing the capability model of the user based on the historical exercise record of the user, as described in the content performed in step 601. In this embodiment of this application, the capability model of the user may be, for example, the running capability model configured to measure the running capability of the user.

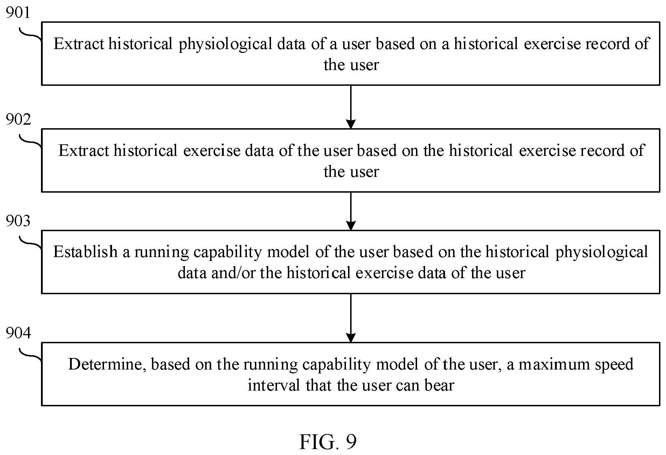

FIG. 9 is a diagram of an implementation procedure of a running capability model for training the user according to this embodiment of this application. It may be understood that a body that performs the steps of the procedure shown in FIG. 9 may be a mobile terminal device on which an application such as a health app is installed, for example, the mobile phone 10 in this embodiment of this application. In the following descriptions of content of each step, a body that performs each step is not described repeatedly.

901: Extract historical physiological data of the user based on a historical exercise record of the user.

For example, the extracted historical physiological data includes but is not limited to a gender, an age, a heart rate reserve (HRR), a maximal heart rate (MaxHR), a rest heart rate (RestHR), a maximal oxygen uptake, and the like of the user.

It may be understood that the historical physiological data of the user is usually stable in a specific period of time, and does not change greatly. However, as the user ages and depending on sustainability of exercise habits of the user, there may be some changes, which may also reflect changes of physical fitness of the user. Therefore, the historical exercise record based on which the historical physiological data is extracted may be a record in the last half year, a record in the last three months, or a record in the last one month, and an extraction result is usually accurate.

902: Extract historical exercise data of the user based on the historical exercise record of the user.

For example, the extracted historical exercise data may include but is not limited to a distance, exercise duration, data related to a heart rate curve, a heart rate fluctuation range, a speed in each time period, and calories burned.

In some embodiments, the historical exercise record based on which the historical exercise data is extracted may be an exercise record in the last three months. In some other embodiments, the historical exercise data may be extracted based on a historical exercise record in the last two months or in the last half a year. This is not specifically limited in this application. In addition, after an exercise record of the user is updated, a historical exercise record three months ago or the like may be correspondingly deleted, so that a historical exercise record used to extract historical exercise data is added to a latest exercise record of the user in a timely manner, to improve a degree of matching between a finally established running capability model and a current actual running capability of the user.

903: Establish the running capability model of the user based on the historical physiological data and/or the historical exercise data of the user.

For example, the running capability model established for the user when a data amount corresponding to the historical exercise data of the user is sufficient may be a correspondence between a running capability (namely, a running capability) of the user, and exercise duration/a calorie burned value/a distance, a speed, an incline, a real-time heart rate, and the like. As an example, for a running capability calculation formula determined by using the running capability model, refer to the following formula (1):

$$V=A*(Time/Cal/Dis)+B*(Speed)+C*(Incline)+D*HR+P \qquad (1).$$

In the formula, V indicates the running capability of the user; Time/Cal/Dis respectively indicate the exercise duration/the calorie burned value/the distance, and are usually corresponding parameter values input by the user for a purpose of exercise; Speed indicates the speed; Incline indicates the incline; and HR indicates the real-time heart rate of the user. A, B, C, and D are coefficients of corresponding physiological parameters, and P is a running capability correction value.

When a data amount corresponding to the historical exercise data is insufficient to establish the running capability model, the mobile phone 10 may also calculate the exercise capability of the user based on the maximal oxygen uptake data in the historical physiological data of the user. The exercise capability is the running capability of the user in this embodiment of this application. For a specific calculation formula for calculating the running capability of the user based on the maximal oxygen uptake, refer to the following formula (2):

$$V=Vo2Max-Z \qquad (2).$$

In the formula, Vo2Max indicates the maximal oxygen uptake of the user. The maximal oxygen uptake may be an average value, a common value, a maximum value, or the like of the maximal oxygen uptake in the historical physiological data of the user. This is not limited herein. Z is a constant, indicating a difference between the maximal oxygen uptake and a running capability value.

When a data amount corresponding to the historical exercise data is insufficient and the maximal oxygen uptake data is also missing, the mobile phone 10 may further calculate the running capability of the user by using a body mass index (body mass index, BMI) of the user. For a specific calculation formula for calculating the running capability of the user by using the body mass index, refer to the following formula (3):

$$V=BMI+Q \qquad (3).$$

In the formula, BMI indicates the body mass index, also known as a body mass index, and is equal to a weight (kilogram) divided by a height (meter) squared; and Q is a constant, and indicates a difference between the running capability of the user and the body mass index.

904: Determine, based on the running capability model of the user, a maximum speed interval that the user can bear.

For example, a speed (Speed) that the user can reach and an incline (Incline) that the user can bear under different control parameters such as the exercise duration, the calorie burned value, and the distance may be calculated based on the running capability model established in step 903 and the correspondingly trained running capability value, and with reference to the foregoing formula (1).

In this way, a corresponding maximum speed interval may be calculated based on a speed distribution range and an incline distribution range that are obtained through calculation.

The maximum speed interval may be determined through calculation according to the following formula (4):

$$Speed\_max\_pct=Speed/Speed\_max \qquad (4).$$

An end value of a speed interval that matches the running capability of the user may be determined through calculation according to the following formula (5):

$$Speed\_pct=(Speed\ max\_pct-G)/H \qquad (5).$$

In the formula, Speed max_pct indicates the maximum speed interval, and G and H are constants measured experimentally.

The following specifically describes a manner of determining an exercise control parameter in each phase based on the training target set by the user and the running capability-related parameter of the user in content performed in step 604.

Table 2

| Training target/Training course | Action phase | Exercise load |
|---|---|---|
| 3 km target running/heart rate-regulated running | Phase 1 | Training type: Training_zone type=$\alpha$;<br>Target distance: Target Dis=a (km); |
| | | Target heart rate and fluctuation range: (Target heart rate) Target HR=R (dpm) (Heart rate zone) Target HR_pct_scope=[HR0, HR1] (Maximal heart rate zone) HR_max_pct=Target HR/HR_max<br>(Heart rate zone) HR_pct=(HR_max_pct-E)/F<br>Target speed and fluctuation range:<br>Target speed: Target speed (Speed interval) Target speed scope=[TS0, TS1]<br>Target incline: Target incline |
| | Phase 2 | ... |
| | Phase 3 | ... |
| | Phase N | ... |

In the foregoing Table 2, for a formula for calculating a minimum heart rate HR0, refer to the following formula (6):

$$HR0=rest\_HR+(max\_HR-rest\_HR)*HR\_pct\_scope\_low \qquad (6).$$

In the formula, rest_HR indicates the rest heart rate, max_HR indicates the maximal heart rate, and HR_pct_scope_low indicates a minimum value in a proportion interval of a corresponding heart rate difference. The proportion interval of the heart rate difference indicated by HR_pct_scope is an experimental value or a training empirical value. For example, if a value of HR_pct_scope is [0.8, 0.85], a value of HR_pct_scope_low is 0.8.

For a formula for calculating the maximal heart rate HR1, refer to the following formula (7):

$$HR1=rest\_HR+(max\_HR-rest\_HR)*HR\_pct\_scope\_high \qquad (7).$$

In the formula, HR_pct_scope _high indicates a maximum value in a proportion interval of a corresponding heart rate difference.

It may be understood that the target heart rate zone (Target HR Scope) may be [HR0, HR1].

If HR1-HR0>K (corresponding to an excessively large heart rate fluctuation range) and or HR1-HR0<L (corresponding to an excessively small fluctuation range), the target heart rate zone (Target HR Scope) may be [(HR0+HR1)/2-10, (HR0+HR1)/2+10].

In addition, in Table 2, for a formula for calculating the target speed (target speed, TS), refer to the following formula (8):

$$TS=b+w1*(HR\_pct)^2+w2*x+w3*(Vdot)^2+\ldots*(Vdot)^2+wN*(HR\_pct)^2*Vdot \qquad (8).$$

In the formula, w1, w2, w3, ..., wN are weight coefficients.

For a formula for calculating a minimum speed value (TS0) in [TS0, TS1] corresponding to the speed interval (Target speed scope), refer to the following formula (9):

$$TS0=b+w1*(HR\_pct–L)^2+w2*x+w3*(Vdot)^2+\ldots*(Vdot)^2+wN*(HR\_pct–L)^2*Vdot \qquad (9).$$

For a formula for calculating the maximum speed (TS1), refer to the following formula (10):

$$TS1=b+w1*(HR\_pct+w)^2+w2*x+w3*(Vdot)^2+\ldots*(Vdot)^2+wN*(HR\_pct+w)^2*Vdot$$

$$(10).$$

In some other embodiments, related parameters such as the target heart rate and fluctuation range, the target speed and fluctuation range, and the like that correspond to each phase in Table 1 may alternatively be calculated in another calculation manner. This is not limited in this application.

The target incline (target incline) may be determined through calculation with reference to a related calculation method in a conventional technology. Details are not described herein.

The following describes in detail a specific process of adjusting the exercise control parameter in the corresponding phase in the training plan based on the real-time physiological parameter in step 609 to step 616.

It may be understood that there are usually the following two occasions in which the treadmill 30 adjusts a load in a corresponding phase based on a load-related parameter in the adjusted exercise control parameter in the training plan:

A first adjustment occasion is when switching between adjacent action phases in the training plan.

A second adjustment occasion is when a training load needs to be adjusted in real time due to a large difference between a heart rate zone of the user and a target heart rate zone in a same action phase.

Therefore, when detecting that a real-time heart rate of the user deviates from a target heart rate zone at a corresponding phase, the mobile phone 10 may adjust the corresponding phase in the training plan at the foregoing two adjustment occasions based on a deviation degree.

It may be understood that, if a difference between a current speed and a target speed in a next phase is large when an exercise phase is to be switched, to avoid discomfort of the user caused by a large speed adjustment, the current speed may be segmentally adjusted to the target speed, that is, the load is segmentally adjusted. In addition, to avoid poor user experience due to frequent adjustment of a pace of the treadmill in a same action phase, a frequency threshold or a time proportion threshold for adjusting the load may be further set. For example, a preset time proportion threshold for segmentally adjusting a current load (for example, a current speed) to a target load (for example, a target speed) is 20%, so that total time for segmentally adjusting the load cannot exceed 20% of total time of a corresponding action phase.

In addition, since the human body's tolerance for increasing a load of an exercise apparatus is generally lower than tolerance for decreasing a load of the exercise apparatus, an adjustment frequency during load increase can be controlled to be higher than an adjustment frequency during load decrease, and an adjustment magnitude during load increase can be less than an adjustment magnitude during load decrease.

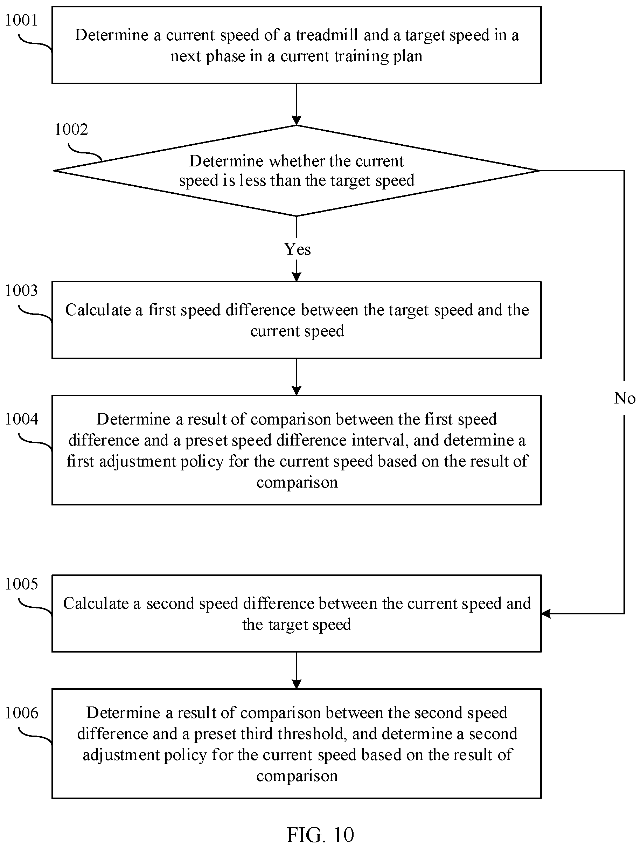

FIG. 10 is a diagram of an implementation procedure of adjusting a load of the treadmill based on collected real-time exercise data according to this embodiment of this application.

It may be understood that, in this embodiment of this application, a body that performs the steps of the adjustment procedure shown in FIG. 10 may be the mobile phone 10. In other words, the mobile phone 10 may adjust the load of the treadmill 30 based on the collected real-time exercise data. The following describes the steps of the adjustment procedure shown in FIG. 10 by using an example in which a correspondingly adjusted load is a speed.

Specifically, the procedure includes the following steps.

1001: Determine a current speed of the treadmill and a target speed in a next phase in a current training plan.

1002: Determine whether the current speed is less than the target speed.

If the current speed is less than the target speed, step 1003 and step 1004 may be performed to adjust a speed load.

If the current speed is greater than the target speed, step 1005 and step 1006 may be performed to adjust a speed load.

It may be understood that, if the current speed is equal to the target speed, the load of the treadmill 30 may not be adjusted, and a pace of the treadmill 30 is kept at the current speed or the target speed.

1003: Calculate a first speed difference between the target speed and the current speed.

1004: Determine a result of comparison between the first speed difference and a preset speed difference interval, and determine a first adjustment policy for the current speed based on the result of comparison.

For example, the preset speed difference interval may be, for example, [m, n], where m indicates a lower limit of the preset speed interval, and n indicates an upper limit of the preset speed interval. Further, the result of comparison between the first speed difference and the preset speed difference interval and the first adjustment policy determined based on the result of comparison may include the following several cases:

> (a) When first speed difference<m, the current speed is increased to the target speed, that is, the load is increased to a target load.
> (b) When m≤first speed difference<n, the current speed is increased to the target speed minus a, that is, the load is increased to a target load-a, where a is a preset difference constant.
> (c) When first speed difference≥n, a segmental adjustment policy for a load of a corresponding exercise apparatus may be determined by determining whether a collected real-time heart rate is within a heart rate zone in a corresponding phase, for example, the pace of the treadmill is adjusted by using a dynamic speed adjustment algorithm. The foregoing dynamic speed adjustment algorithm executed based on the collected real-time heart rate is described in detail below. Details are not described herein again.

For example, the lower limit m of the preset speed difference interval may correspond to the first threshold in step 609, for example, may be preset as a speed difference such as 1.2 km/h. The upper limit n of the preset speed difference interval may correspond to the second threshold in step 610, for example, may be preset as a speed difference such as 2.5 km/h. The difference constant a used to control an adjustment amplitude may be preset as a speed constant such as 1 km/h.

1005: Calculate a second speed difference between the current speed and the target speed.

1006: Determine a result of comparison between the second speed difference and a preset third threshold, and determine a second adjustment policy for the current speed based on the result of comparison.

For example, if the current speed is greater than the target speed, it indicates that a load of a corresponding exercise apparatus needs to be decreased. In this case, whether to perform segmental adjustment may be controlled by using a preset large speed difference threshold, for example, the third threshold. A specific adjustment process may include, for example, the following several cases:

> 1. When second speed difference≤k, the current speed is decreased to the target speed, that is, the load is decreased to a target load.
> 2. When second speed difference>k, a segmental adjustment policy for a load of a corresponding exercise apparatus may be determined by determining whether a collected real-time heart rate is within a target heart rate zone in a corresponding phase, for example, the pace of the treadmill is adjusted by using a dynamic speed adjustment algorithm. The foregoing dynamic speed adjustment algorithm executed based on the collected real-time heart rate is described in detail below. Details are not described herein again.

For example, the preset third threshold k may be preset to a speed difference such as 3 km/h. This is not limited herein. In addition, it may be understood that, in some embodiments, the third threshold may be greater than the second threshold. In some other embodiments, the third threshold may alternatively be equal to the second threshold. This is not limited herein.

The following further specifically describes a specific implementation process of the dynamic speed adjustment algorithm executed based on the collected real-time heart rate.

As described above, in an exercise process of the user, if a target speed in a next action phase that is to be performed by the user is significantly higher than the current speed, for example, the case (c) in which the first speed difference is greater than 2.5 in step 1004, or the case 2 in which the second speed difference is greater than 3 in step 1006, in this case, to avoid discomfort of the user caused by large adjustment at a time, load adjustment may be segmentally controlled. For example, a load of an exercise apparatus may be dynamically adjusted by monitoring a real-time heart rate of the user. It may be understood that the current speed is a current pace used by the user on the treadmill 30, and is also a running speed of the user in a current action phase.

Specifically, a segmental adjustment policy for a corresponding load may be dynamically determined by determining whether a real-time heart rate of the user collected through real-time monitoring is in a target heart rate zone corresponding to a corresponding action phase. As an example, the policy may include the following two types:

**Policy 1:** a dynamic heart rate adjustment policy, that is, whether the load of the exercise apparatus needs to be adjusted is determined based on whether the real-time heart rate is within the target heart rate zone. In addition, according to the policy 1, it may be further determined, based on a difference between an upper limit and a lower limit of the real-time heart rate, a size in which the load needs to be increased or decreased.

It may be understood that, according to the policy 1, the user needs to wear a wearable device throughout the process. For example, the user needs to wear the watch 20 in an exercise process to collect the real-time heart rate. In addition, in a process of sampling the real-time heart rate data, the wearable device such as the watch 20 worn by the user should ensure that the collected real-time heart rate data is valid heart rate data. The collected real-time heart rate data may be determined by determining whether a proportion of valid data is greater than a first preset proportion threshold (or referred to as a normal proportion), or may be determined by determining whether a proportion of invalid data is less than a second preset proportion threshold (or referred to as an abnormal proportion). This is not limited herein.

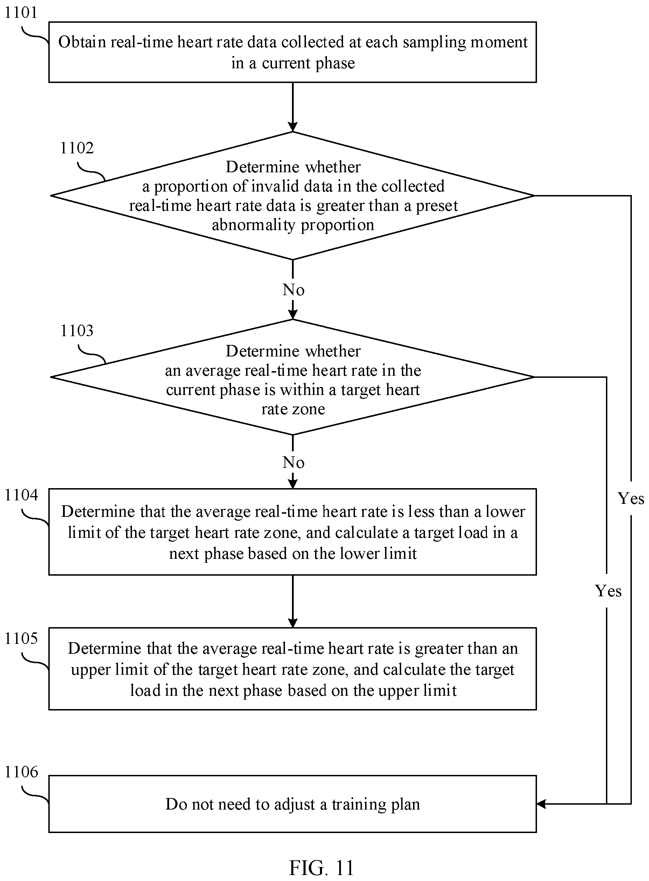

For example, FIG. 11 is a diagram of an implementation procedure of adjusting a load of the treadmill based on a real-time heart rate according to this embodiment of this application.

It may be understood that, in this embodiment of this application, a body that performs the steps of the adjustment procedure shown in FIG. 11 may be the mobile phone 10. That is, the mobile phone 10 may adjust the load of the treadmill 30 based on the collected real-time exercise data and physiological data. The following describes the steps of the adjustment procedure shown in FIG. 11 by using an example in which a correspondingly adjusted load is a speed/an incline.

As shown in FIG. 11, the procedure includes the following steps.

1101: Obtain real-time heart rate data collected at each sampling moment in a current phase.

1102: Determine whether a proportion of invalid data in the collected real-time heart rate data is greater than a preset abnormality proportion.

If a determining result is that the proportion of the invalid data is greater than the preset abnormality proportion, it indicates that there is a large amount of invalid data in the collected real-time heart rate data, and the collected real-time heart rate data cannot be used to determine whether the load is adjusted. In this case, step 1106 may be performed without a need to adjust a training plan. In addition, a corresponding interface of the mobile phone 10 may further display a prompt message, for example, a prompt message "Your effective heart rate cannot be collected currently. Please check whether the wearable device is properly worn".

If a determining result is that the proportion of the invalid data is not greater than the preset abnormality proportion, it indicates that valid data in the collected real-time heart rate data is sufficient for adjusting and determining whether the load is to be adjusted. In this case, step 1103 may continue to be performed to further determine whether the collected real-time heart rate is within a target heart rate zone.

1103: Determine whether an average real-time heart rate in the current phase is within the target heart rate zone.

If a determining result is that the average real-time heart rate in the current phase is within the target heart rate zone, it indicates that the real-time heart rate of the user can adapt to a load in the current phase, and a current training plan can also match a current exercise capability of the user. In this case, step 1106 may be performed without a need to adjust a load control parameter of a corresponding exercise apparatus in the training plan.

If a determining result is that the average real-time heart rate at the current phase is not within the target heart rate zone, it indicates that the real-time heart rate of the user may not adapt to a load in the current phase, and step 1104 and step 1105 need to be performed, to adjust the load of the corresponding exercise apparatus based on a result of comparison between the average real-time heart rate and, an upper limit and a lower limit of the target heart rate zone.

For example, the target heart rate zone may be a recommended heart rate zone that is correspondingly set in the current phase. For example, the target heart rate zone may be [70, 210), that is, $70 \leq HRavg$ (average real-time heart rate) $<210$. When the average real-time heart rate obtained through calculation is within [70, 210), the determining result is that the average real-time heart rate at the current phase is within the target heart rate zone; otherwise, the determining result is that the average real-time heart rate at the current phase is not within the target heart rate zone.

In some other embodiments, the average real-time heart rate obtained through calculation may be an arithmetic average value, a geometric average value, a harmonic average value, or the like. This is not limited herein.

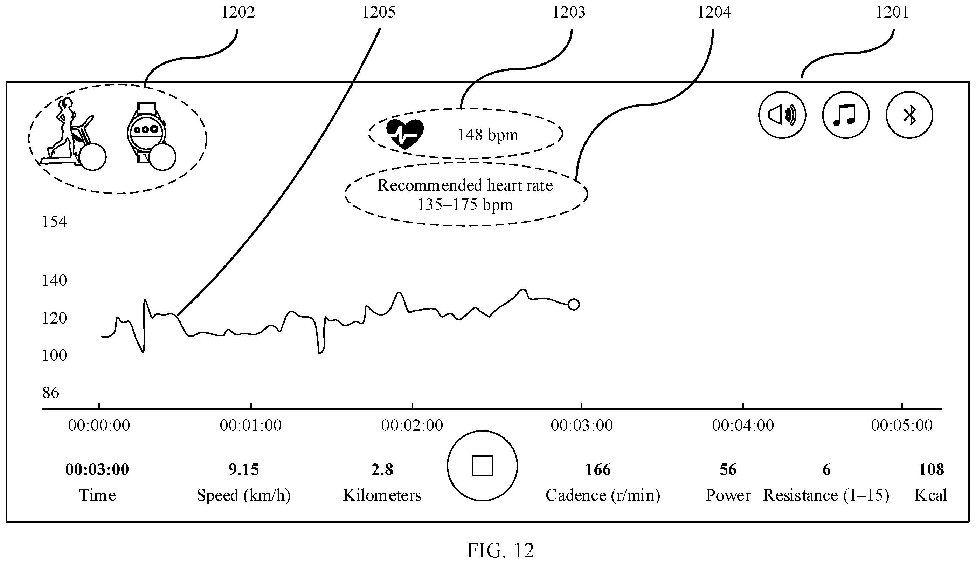

For example, FIG. 12 is a diagram of an exercise interface correspondingly displayed in an exercise process of the user according to this embodiment of this application.

As shown in FIG. 12, information such as an indication icon 1202 indicating that the watch 20 and the treadmill 30 are connected, a display area 1203 for displaying a collected real-time heart rate, and a display area 1204 for displaying a recommended heart rate may be displayed on the exercise interface 1201 displayed by the mobile phone 10. The recommended heart rate is, for example, a target heart rate zone corresponding to a current phase. The exercise interface 1201 may further display a real-time heart rate curve 1205 of the user that changes with time, which may be referred to as a heart rate curve for short in this embodiment of this application.

Therefore, the foregoing average real-time heart rate that needs to be calculated in the current phase may be obtained by calculating an average value of real-time heart rate data collected within exercise duration of the user in the current phase, for example, calculating an average value of real-time heart rate data that corresponds to each moment, collected within 3 minutes, and displayed in the heart rate curve 1205 shown in FIG. 12. In some other embodiments, the exercise interface shown in FIG. 12 may alternatively display an average real-time heart rate obtained through real-time calculation. This is not limited herein.

1104: Determine that the average real-time heart rate is less than a lower limit of the target heart rate zone, and calculate a target load in a next phase based on the lower limit.

For example, in the foregoing example, if the average real-time heart rate in the current phase that is obtained calculation falls outside the target heart rate zone and HR<70 beats/minute, the target load in the next phase may be calculated based on the target heart rate=70 beats/minute. The target load may be a target speed in this embodiment of this application. In this case, it may be determined that a heart rate of the user is low. In this embodiment of this application, to increase the heart rate of the user, adjustment may be performed by increasing a load, for example, in an acceleration manner. In some embodiments, the target load obtained through calculation based on the upper limit of the target heart rate zone may be higher than a target load originally designed in this phase. This is not limited herein.

It may be understood that, in the acceleration process, a manner of calculating a target speed and speed duration in the next phase may be implemented by using the following relational expressions (11) and (12):

$$\text{Target speed in the next phase (TS)} = \text{Larger round-up (Constant 5*Current speed+Constant 6*Target speed} + \text{Constant 7, 1)} \tag{11}$$

$$\text{Speed duration} = \text{Minimum round-up (Constant 1*Current speed+Constant 2*Target speed+Constant 3*Current speed square+Constant 4*Target speed square+Constant, 0)} \tag{12}$$

1105: Determine that the average real-time heart rate is greater than the upper limit of the target heart rate zone, and calculate the target load in the next phase based on the upper limit.

For example, in the foregoing example, if the average real-time heart rate in the current phase that is obtained calculation falls outside the target heart rate zone and HR>210 beats/minute, the target load in the next phase may be calculated based on the target heart rate=210. The target load may be a target speed in this embodiment of this application. In this case, it may be determined that a heart rate of the user is high. In this embodiment of this application, to increase the heart rate of the user, adjustment may be performed by decreasing a load, for example, in a deceleration manner. In some embodiments, the target load obtained through calculation based on the lower limit of the target heart rate zone may be lower than a target load originally designed in this phase. This is not limited herein.

It may be understood that, in the deceleration process, a manner of calculating a target speed and speed duration in the next phase may be implemented by using the following relational expressions (13) and (14):

$$\text{Target speed in the next phase (TS)} = \text{Larger round-up (Constant 8*Current speed+Constant 9*Target speed} + \text{Constant 10) (1)} \tag{13}$$

$$\text{Duration} = \text{Minimum round-up (Constant 11*Current speed+Constant 12*Target speed+Constant 13*Current speed square+Constant 14*Target speed square+Constant, 0)} \tag{14}$$

1106: Do not need to adjust the training plan does not need to be adjusted.

It may be understood that an advantage of the policy 1 is that a training plan of a corresponding user may be adjusted in real time with reference to a physiological status of the user of the day.

In some other embodiments, another policy, for example, the following policy 2, may also be used in a process of adjusting the training plan based on a heart rate change of the user. This is not limited herein.

**Policy 2:** a static heart rate adjustment policy, that is, adjustment is performed based on a fixed step value and a relationship between a load increase and heart rate stabilization time in a running capability model of the user. Details are not described herein again in this application.

An advantage of the policy 2 is that, on a basis that the running capability model of the user has been established, the user does not need to wear a watch throughout the process. In addition, a load increase and decrease are more specific, and user experience is not affected by frequent increase and decrease of a load in segments.

With reference to another Embodiment 2, the following describes a specific implementation process of the exercise control solution provided in this application in the cycling exercise scenario.

**Embodiment 2**

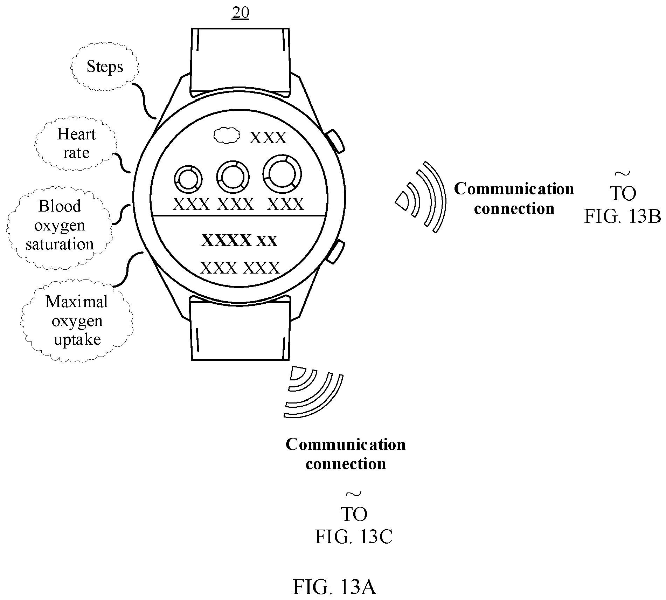

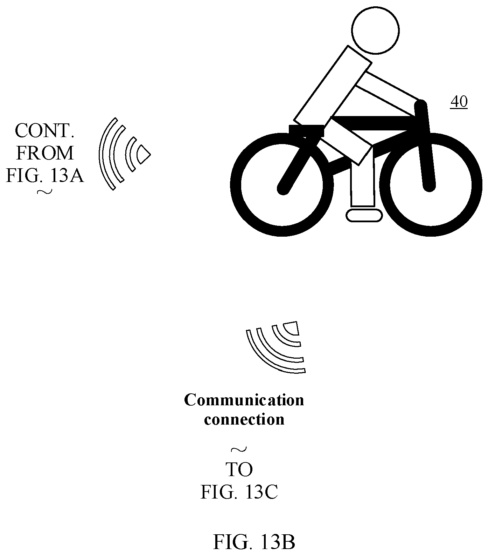

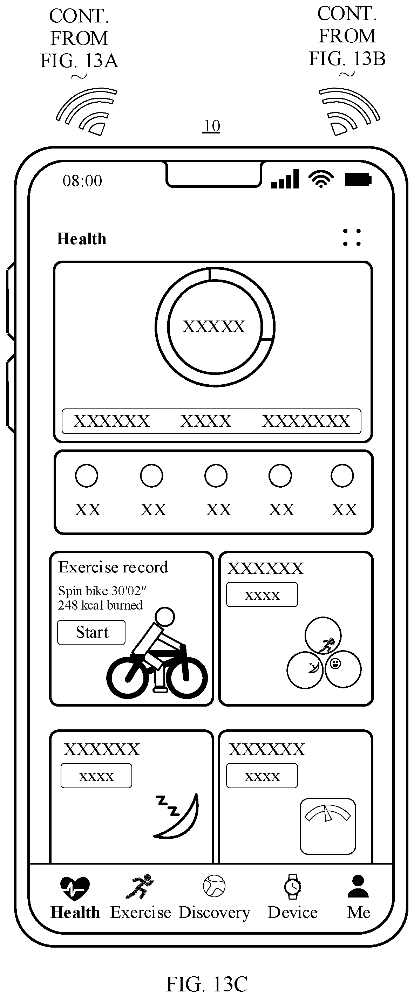

FIG. 13A to FIG. 13C are a diagram of a cycling exercise scenario according to an embodiment of this application.

As shown in FIG. 13A to FIG. 13C, the scenario may include a mobile phone 10, a watch 20, and a spin bike 40 that establish communication connections to each other. The spin bike 40 is a resistance-type exercise apparatus. In some other embodiments, the exercise apparatus in the scenario shown in FIG. 13A to FIG. 13C may alternatively be another speed-type exercise apparatus, for example, an elliptical trainer. This is not limited herein.

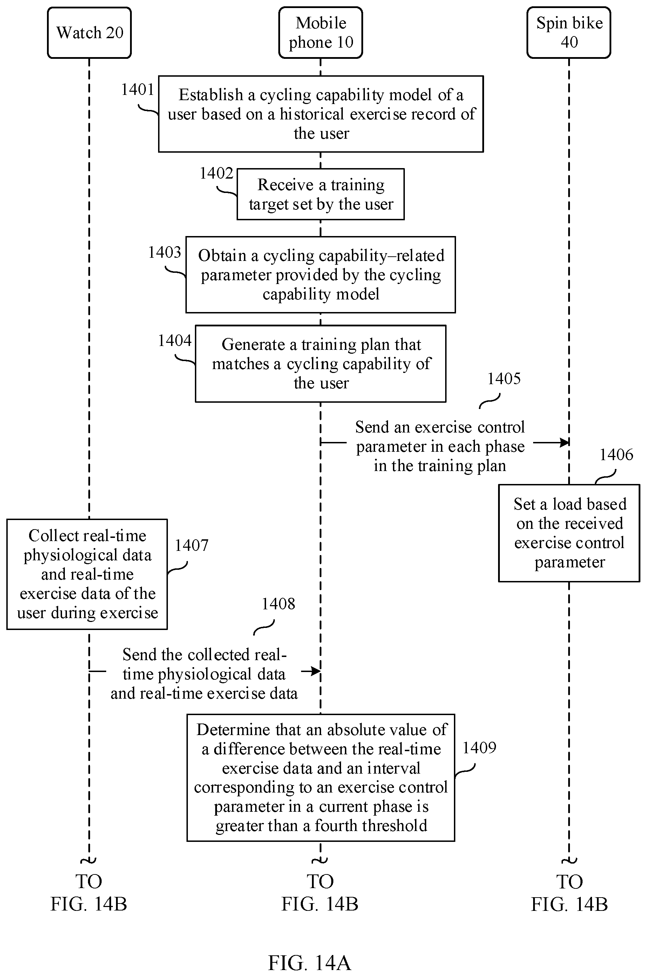

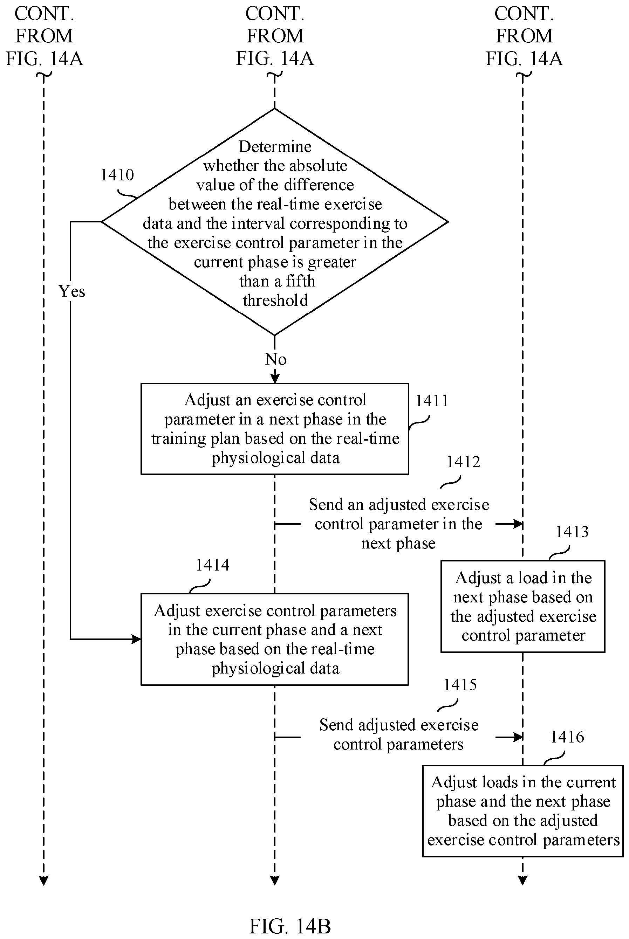

FIG. 14A and FIG. 14B are a diagram of an implementation procedure of another exercise control method according to an embodiment of this application. The implementation procedure is an interaction procedure implemented by the mobile phone 10, the watch 20, and the spin bike 40 that perform each step.

1401: The mobile phone 10 establishes a cycling capability model of a user based on a historical exercise record of the user.

For example, for a related process in which the mobile phone 10 establishes the capability model based on the historical exercise record of the user, refer to related descriptions in step 601 in Embodiment 1. Different from content performed in step 601 in Embodiment 1, in this embodiment of this application, the established capability model is a cycling capability model.

For a specific process of establishing the cycling capability model, refer to the procedure shown in FIG. 9 in Embodiment 1 and related descriptions. Details are not described herein again. The cycling capability model Q established based on the historical exercise record of the user may reflect, to some extent, a correspondence between a cycling capability of the user and exercise duration/a calorie burned value/a distance, a power, a real-time heart rate, and the like. As an example, for a running capability calculation formula determined by using the cycling capability model, refer to the following formula (15):

$$Q=A*(Time/Cal/Dis)+B*(Power)+C*(Incline)+D*HR+P \qquad (15).$$

In the formula, Q indicates the cycling capability of the user; Time/Cal/Dis respectively indicate the exercise duration/the calorie burned value/the distance, and are usually corresponding parameter values input by the user for a purpose of exercise; Power indicates the speed; Incline indicates the incline; and HR indicates the real-time heart rate of the user. A, B, C, and D are coefficients of corresponding physiological parameters, and P is a cycling capability correction value.

When a data amount corresponding to the historical exercise data is insufficient to establish the cycling capability model, the mobile phone 10 may also calculate an exercise capability of the user based on maximal oxygen uptake data in historical physiological data of the user. The exercise capability is the cycling capability of the user in this embodiment of this application. For a specific calculation formula for calculating the cycling capability of the user by using the maximal oxygen uptake, refer to the following formula (16):

$$Q=Vo2max*Weight*q \text{ (coefficient)} \qquad (16).$$

In the formula, Vo2Max indicates the maximal oxygen uptake of the user. The maximal oxygen uptake may be an average value, a common value, a maximum value, or the like of the maximal oxygen uptake in the historical physiological data of the user. This is not limited herein. Weight indicates a weight of a user and q indicates a weight coefficient.

When a data amount corresponding to the historical exercise data is insufficient and the maximal oxygen uptake data is also missing, the cycling capability Q of the user may be a constant. The constant is a constant determined through statistics collection based on experimental data, for example, Q=1.1. This is not limited herein.

1402: The mobile phone 10 receives a training target set by the user.

For example, the user may set the training target on a related exercise control interface displayed by the mobile phone 10. Different from content performed in step 602 in Embodiment 1, in this embodiment of this application, the training target set by the user may be a distance target, a time target, a calorie burning target, or the like. For a specific interface and operation manner for setting the training target, refer to related descriptions in step 602 in Embodiment 1 and related interface examples. Details are not described herein again.

1403: The mobile phone 10 obtains a cycling capability-related parameter provided by the cycling capability model.

Different from content performed in step 603 in Embodiment 1, in this embodiment of this application, the mobile phone 10 obtains the cycling capability-related parameter provided by the cycling capability model of the user. In some embodiments, a running capability model, the cycling capability model, and the like of the user may also be unified as a comprehensive exercise capability model of the user. This is not limited herein.

For a specific process in which the mobile phone 10 obtains the cycling capability-related parameter provided by the cycling capability model, refer to related descriptions of a process of obtaining the running capability-related parameter in step 603 in Embodiment 1. Details are not described herein again.

1404: The mobile phone 10 generates a training plan that matches the cycling capability of the user.

For example, the mobile phone 10 may generate, based on the training target set by the user and with reference to the cycling capability-related parameter provided by the cycling capability model of the user, the training plan that matches the cycling capability of the user. The training plan may include exercise control parameters in a plurality of phases, and an exercise control parameter in each phase is a related parameter that matches the cycling capability of the user.

For example, as shown in Table 3, the training plan may include a training target/training course, action phases 1 to N, an exercise control parameter corresponding to each phase, and the like.

Table 3

| Training target/Training course | Action phase | Exercise load |
|---|---|---|
| Three-kilometer cycling | Phase 1 | Training type: Training_zone type=$\alpha$;<br>Target distance: Target Dis=a (km);<br>Target heart rate and fluctuation range: (Target heart rate) Target HR=R (dpm) (Heart rate zone) Target HR_pct_scope=[HR0, HR1] (Maximal heart rate zone) HR_max_pct=Target HR/HR_max<br>(Heart rate zone) HR_pct=(HR_max_pct-E)/F<br>Target power and fluctuation range:<br>Target power: Target power (Power interval) Target power scope=[TP0, TP1] |
| | Phase 2 | ... |
| | Phase 3 | ... |
| | Phase N | ... |

In Table 3, for a formula for calculating a minimum heart rate HR0, refer to the formula (6); and for a formula for calculating a maximal heart rate HR1, refer to the formula (7).

In addition, in Table 3, for a formula for calculating the target power (target power, TP), refer to the following formula (17):

$$TP=b+w1*(HR\_pct)^2+w2*x+w3*(Vdot)^2+\ldots*(Vdot)^2+wN*(HR\_pct)^2*Vdot \qquad (17).$$

In the formula, w1, w2, w3, ..., wN are weight coefficients.

For a formula for calculating a minimum power (TP0) in [TP0, TP1] corresponding to the power interval (target power scope), refer to the following formula (18):

$$TP0=b+w1*(HR\_pct–L)^2+w2*x+w3*(Vdot)^2+\ldots*(Vdot)^2+wN*(HR\_pct–L)^2*Vdot$$

$$(18).$$

For a formula for calculating a maximum power (TP1), refer to the following formula (19):

$$TP1=b+w1*(HR\_pct+w)^2+w2*x+w3*(Vdot)^2+\ldots*(Vdot)^2+wN*(HR\_pct+w)^2*Vdot$$

$$(19).$$

In some other embodiments, related parameters such as the target heart rate and fluctuation range, the target power and fluctuation range, and the like that correspond to each phase in Table 3 may alternatively be calculated in another calculation manner. This is not limited in this application.

1405: The mobile phone 10 sends the exercise control parameter in each phase in the training plan to the spin bike 40.

Different from content performed in step 605 in Embodiment 1, in this embodiment of this application, the exercise control parameter in each phase in the training plan sent by the mobile phone 10 is a cycling capability-related parameter, and is sent to the spin bike 40.

For a specific process in which the mobile phone 10 sends the exercise control parameter in each phase to the spin bike 40, refer to related descriptions of the process in which the exercise control parameter in each phase is sent to the treadmill 30 in step 605 in Embodiment 1. Details are not described herein again.

1406: The spin bike 40 sets a load based on the received exercise control parameter.

Different from content performed in step 606 in Embodiment 1, in this embodiment of this application, an exercise apparatus whose load is set is the spin bike 40. Therefore, the set load corresponding to the spin bike 40 may be a power (power) of the spin bike, which is referred to as power for short below.

Power P of spin bike=RPM (cycling speed of user)*R (resistance gear)*n (fixed coefficient of resistance system of spin bike). In some other embodiments, the power of the spin bike may alternatively be calculated in another manner. This is not limited herein.

For a specific process in which the spin bike 40 sets the load based on the received exercise control parameter, refer to related descriptions of the process in which the treadmill 30 sets a load in step 606 in Embodiment 1. Details are not described herein again.

1407: The watch 20 collects real-time physiological data and real-time exercise data of the user during exercise.

1408: The watch 20 sends the collected real-time physiological data and real-time exercise data to the mobile phone 10.

Execution content of step 1407 and step 1408 is the same as that of step 607 and step 608 in Embodiment 1. For details, refer to related descriptions in step 607 and step 608 in Embodiment 1. Details are not described herein.

1409: The mobile phone 10 determines that an absolute value of a difference between the real-time exercise data and an interval corresponding to an exercise control parameter in a current phase is greater than a fourth threshold.

1410: The mobile phone 10 determines whether the absolute value of the difference between the real-time exercise data and the interval corresponding to the exercise control parameter in the current phase is greater than a fifth threshold, where the fifth threshold is greater than the fourth threshold.

1411: The mobile phone 10 adjusts an exercise control parameter in a next phase in the training plan based on the real-time physiological data.

1412: The mobile phone 10 sends an adjusted exercise control parameter in the next phase to the spin bike 40.

1413: The spin bike 40 adjusts a load in the next phase based on the adjusted exercise control parameter.

1414: The mobile phone 10 adjusts exercise control parameters in the current phase and a next phase based on the real-time physiological data.

1415: The mobile phone 10 sends adjusted exercise control parameters to the spin bike 40.

1416: The spin bike 40 adjusts loads in the current phase and the next phase based on the adjusted exercise control parameters.

Different from content performed in steps 609 to 616 in Embodiment 1, in this embodiment of this application, an object exercise apparatus whose load is adjusted is the spin bike 40. In addition, in this embodiment of this application, before whether the load needs to be adjusted based on the real-time physiological data is determined, whether an absolute value of a difference between a current power and a target power of the spin bike 40 is greater than a corresponding threshold, for example, the fourth threshold or the fifth threshold, may be first determined.

For a process in which the mobile phone 10 adjusts the load of the spin bike 40 based on the real-time physiological data, refer to related descriptions of steps 611 to 616 in Embodiment 1, and steps and related descriptions of the procedure shown in FIG. 11 in Embodiment 1. Details are not described herein again.

Because the spin bike is usually not power supported, actual pedaling feeling of a person is generally affected by a change in resistance. People's active pedaling generates power, and the spin bike can accept a large resistance change. Based on the foregoing relational expression: power P of spin bike=RPM (cycling speed of user)*R (resistance gear)*n (fixed coefficient of resistance system of spin bike), the resistance change may be converted into a power load of the spin bike.

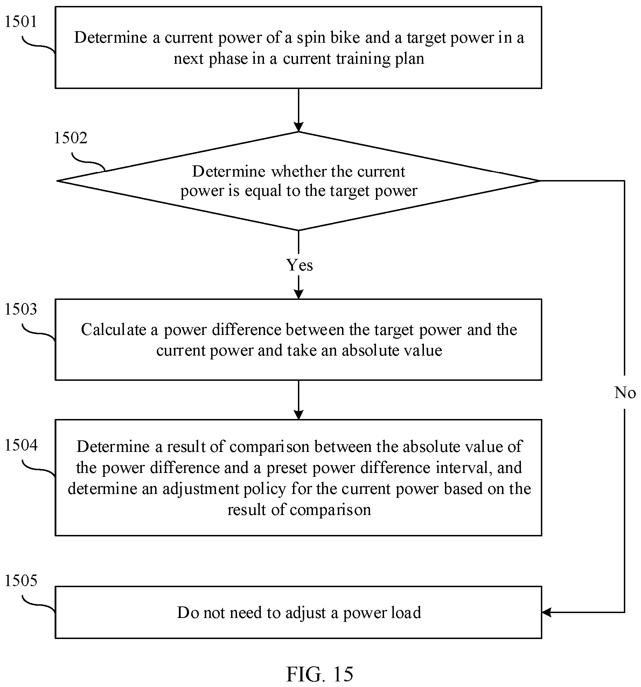

With reference to FIG. 15, the following specifically describes an implementation procedure of adjusting a load of a spin bike based on collected real-time exercise data in an embodiment of this application.

FIG. 15 is a diagram of an implementation procedure of adjusting the load of the spin bike based on the collected real-time exercise data according to this embodiment of this application.

It may be understood that, in this embodiment of this application, a body that performs the steps of the adjustment procedure shown in FIG. 15 may be the mobile phone 10. In other words, the mobile phone 10 may adjust the load of the spin bike 40 based on the collected real-time exercise data. The following describes the steps of the adjustment procedure shown in FIG. 15 by using an example in which the corresponding adjusted load is a power.

Specifically, the procedure includes the following steps.

1501: Determine a current power of the spin bike and a target power in a next phase in a current training plan.

1502: Determine whether the current power is equal to the target power.

If the current power is equal to the target power, step 1505 may be performed. Load adjustment may not be performed on the spin bike. Neither a power load in a current phase nor a power load in the next phase may be adjusted. This is not limited herein.

If the current power is not equal to the target power, step 1503 and step 1504 may be performed to adjust a power load.

1503: Calculate a power difference between the target power and the current power and take an absolute value.

1504: Determine a result of comparison between the absolute value of the power difference and a preset power difference interval, and determine an adjustment policy for the current power based on the result of comparison.

For example, the preset power difference interval may be [i, j], where i indicates a lower limit of the preset power interval, and j indicates an upper limit of the preset power interval. Further, the result of comparison between the absolute value of the power difference and the preset power difference interval and the adjustment policy determined based on the result of comparison may include the following several cases:

(a) When absolute value of power difference<i, the current power may be maintained for first preset duration.

(b) When i≤absolute value of power difference<j, the current power may be maintained for second preset duration. The second preset duration is greater than the first preset duration.

(c) When absolute value of power difference≥j, a segmental adjustment policy for a load of a corresponding exercise apparatus may be determined by determining whether a collected real-time heart rate is within a heart rate zone in a corresponding phase, for example, the power of the spin bike is adjusted by using a dynamic speed adjustment algorithm. In some embodiments, when absolute value of power difference≥j, the current power may be maintained for third preset duration.

For example, the lower limit i of the preset power difference interval may correspond to the fourth threshold in step 1409, for example, may be preset as a power difference such as 20 Hz. The first preset duration may be, for example, 30s. The upper limit j of the preset power difference interval may correspond to the fifth threshold in step 1410, for example, may be preset as a power difference such as 35 Hz. The second preset duration may be duration, for example, 45s, and the third preset duration may be duration, for example, 60s.

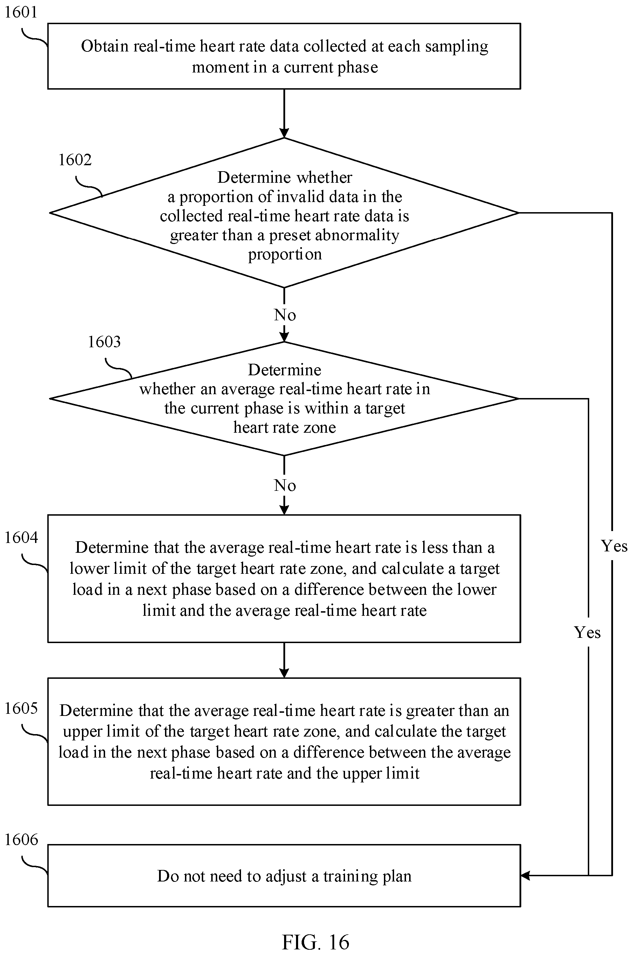

With reference to FIG. 16, the following further describes an execution process of the dynamic speed adjustment algorithm executed based on the collected real-time heart rate.

FIG. 16 is a diagram of an implementation procedure of adjusting the load of the spin bike based on the real-time heart rate according to this embodiment of this application.

It may be understood that, in this embodiment of this application, a body that performs the steps of the adjustment procedure shown in FIG. 16 may be the mobile phone 10. That is, the mobile phone 10 may adjust the load of the spin bike 40 based on the collected real-time exercise data and physiological data. When the steps of the adjustment procedure shown in FIG. 16 are described below, an example in which the corresponding adjusted load is the power of the spin bike is used for description.

Specifically, the procedure includes the following steps.

1601: Obtain real-time heart rate data collected at each sampling moment in a current phase.

1602: Determine whether a proportion of invalid data in the collected real-time heart rate data is greater than a preset abnormality proportion.

If a determining result is that the proportion of the invalid data is greater than the preset abnormality proportion, it indicates that there is a large amount of invalid data in the collected real-time heart rate data, and step 1606 may be performed without a need to adjust the training plan.

If a determining result is that the proportion of the invalid data is not greater than the preset abnormality proportion, it indicates that valid data in the collected real-time heart rate data is sufficient for adjusting and determining whether the load is to be adjusted. In this case, step 1603 may continue to be performed to further determine whether the collected real-time heart rate is within a target heart rate zone.

1603: Determine whether an average real-time heart rate in the current phase is within the target heart rate zone.

If a determining result is that the average real-time heart rate in the current phase is within the target heart rate zone, it indicates that the real-time heart rate of the user can adapt to a load in the current phase, and a current training plan can also match a current exercise capability of the user. In this case, step 1606 may be performed without a need to adjust the training plan.

If a determining result is that the average real-time heart rate in the current phase is not within the target heart rate zone, it indicates that the real-time heart rate of the user may not adapt to a load in the current phase, and step 1604 and step 1605 need to be performed to adjust the load of the corresponding exercise apparatus.

Execution content of step 1601 to step 1603 is the same as that of step 1101 to step 1103 in Embodiment 1. For a specific execution process, refer to related descriptions in step 1101 to step 1103. Details are not described herein again.

1604: Determine that the average real-time heart rate is less than a lower limit of the target heart rate zone, and calculate a target load in a next phase based on a difference between the lower limit and the average real-time heart rate.

For example, in the foregoing example, if the average real-time heart rate in the current phase that is obtained calculation falls outside the target heart rate zone and HR<70 beats/minute, the target load in the next phase may be calculated based on a difference obtained through calculation based on 70-HR. In this embodiment of this application, the target load may be a target power and power holding duration. In this case, it may be determined that a heart rate of the user is low. In this embodiment of this application, to increase the heart rate of the user, adjustment may be performed by increasing a load, for example, increasing a power and/or prolonging duration of a corresponding power.

As an example, specifically, a change amount of a target power in the next phase may be calculated by using the following formula (20):

$$changePower0=min(50, diffHR*Q) \qquad (20).$$

In the formula, changePower0 indicates the change amount of the target power; diffHR indicates a difference between the lower limit of the target heart rate zone and the average real-time heart rate, for example, (70-HR); and Q indicates a cycling capability of the user, and may be obtained through calculation by using the foregoing formula (15), formula (16), or the like. Therefore, the formula (20) indicates that a minimum value selected from a preset minimum power constant (for example, 50 or another value) and a product of a heart rate difference and the cycling capability is used as a target power increment in the next phase, that is, the adjusted target power in the next phase plus the changePower0 is used as an adjusted target power.

1605: Determine that the average real-time heart rate is greater than an upper limit of the target heart rate zone, and calculate the target load in the next phase based on a difference between the average real-time heart rate and the upper limit.

For example, in the foregoing example, if the average real-time heart rate in the current phase that is obtained calculation falls outside the target heart rate zone and HR>210 beats/minute, the target load in the next phase may be calculated based on a difference obtained through calculation based on HR-210. In this embodiment of this application, the target load may be a target power and power holding duration. In this case, it may be determined that a heart rate of the user is high. In this embodiment of this application, to increase the heart rate of the user, adjustment may be performed by decreasing a load, for example, decreasing a power and/or shortening duration of a corresponding power.

As an example, specifically, a change amount of a target power in the next phase may be calculated by using the following formula (21):

$$changePower1=max(100, diffHR*Q) \qquad (21).$$

In the formula, changePower1 indicates the change amount of the target power; diffHR indicates a difference between the average real-time heart rate and the upper limit of the target heart rate zone, for example, (HR-210); and Q indicates a cycling capability of the user, and may be obtained through calculation by using the foregoing formula (15), formula (16), or the like. Therefore, the formula (21) indicates that a maximum value selected from a preset maximum power constant (for example, 100 or another value) and a product of a heart rate difference and the cycling capability is used as a target power decrement in the next phase, that is, the adjusted target power in the next phase minus the changePower1 is used as an adjusted target power.

It may be understood that, in some other embodiments, a manner of adjusting the load of the spin bike based on real-time physiological data such as a real-time heart rate may alternatively be another manner. This is not limited herein.

1606: Do not need to adjust the training plan.

In addition, in this embodiment of this application, a manner of determining a resistance level of the spin bike by combining revolutions per minute (revolutions per minute, RPM) during cycling of the user and a power (power) may be used to implement precise resistance adjustment on the spin bike. In this way, when resistance adjustment is triggered, RPM information needs to be obtained.

In addition, when a first action phase in the training plan is entered, RPM may be excessively low because the user does not perform pedaling, slowly starts pedaling, or the like. In this case, the RPM can be set to 60 by default.

In another action phase, after a power (power) in a corresponding phase is calculated, a value of the RPM may be a value of real-time RPM reported by the spin bike 40 for parameter transfer. However, because the RPM is formed based on the user's pedaling, when the RPM is low, the power remains unchanged. In this case, a resistance level is high, and correspondingly, the user's pedaling is also more difficult. Therefore, the RPM needs to be effectively driven, for example, a recommended RPM range matching a corresponding action phase is preset, and resistance is adjusted within the range.

In some other embodiments, regulation may alternatively be performed by setting a lowest cadence. In this way, when a real-time cadence of the user is less than the lowest cadence, the user may be notified.

For example, the lowest cadence may be set to lower_limit_RPM=50 rmp. In this case, if a cadence of the user in a corresponding action phase is lower than the lowest cadence, the user may be reminded that "Your cadence is excessively low. To achieve a better exercise effect, please keep the cadence above 50 rmp" or other content.

Correspondingly, a limit power corresponding to the cadence (referred to as a cadence limiting power for short) may be obtained through calculation in the following manner:
In a first manner, a cadence correlation coefficient r is calculated according to whether there is a maximal oxygen uptake parameter.

Specifically, when Vo2Max exists, r=min(32, 15*user.vo2max*user.weight/(45*65))

When there is no Vo2Max, r=16.

In a second manner, a cadence limit power is directly calculated based on a preset value of r. For a corresponding algorithm, refer to the following formula (22):

RPM_limit_power=max(20, round(-2.69171183*r+0.16503129*current_RPM-0.00830111*r**2 +0.00525906*currentRPM*#*2+0.19709128*r*currentRPM+1.65261776753 669, 0)) (22).

In the formula, RPM _limit_power indicates the cadence limit power, and the formula (22) indicates that a maximum value of 20 and a calculation result of the function round is used.

It may be understood that, in some other embodiments, the manner of calculating the cadence limit power may alternatively be another manner. This is not limited herein.

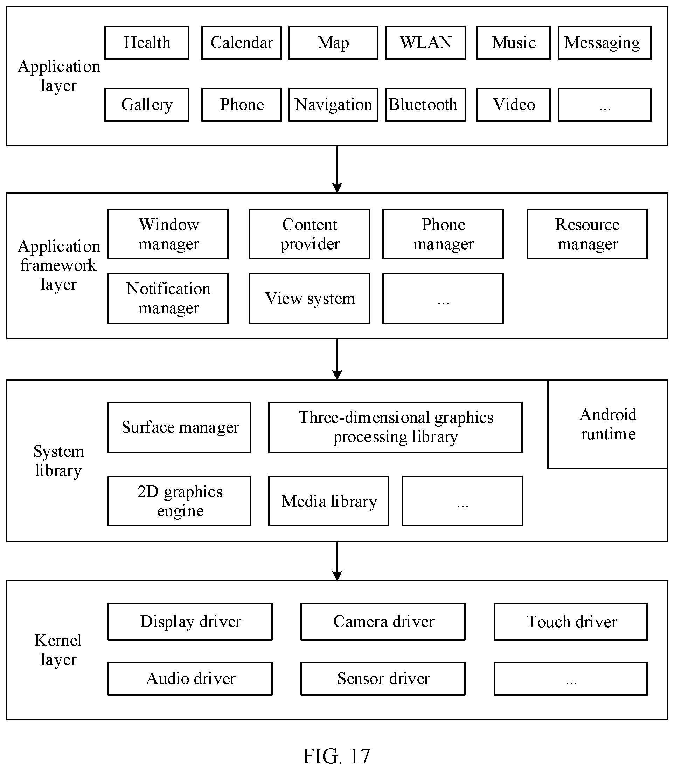

FIG. 17 is a block diagram of a structure of a software system of an electronic device 100 according to an embodiment of this application.

A software system of the electronic device 100 may use a layered architecture, an event-driven architecture, a microkernel architecture, a micro service architecture, or a cloud architecture. In this embodiment of the present invention, an Android system of a layered architecture is used as an example to illustrate the software structure of the electronic device 100. In this embodiment of this application, the electronic device 100 may be an electronic device such as the mobile phone. In some other embodiments, the electronic device 100 may alternatively be a wearable device such as a watch. This is not limited herein.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

As shown in FIG. 17, the application layer may include a series of application packages. The application packages may include applications such as Health, Gallery, Calendar, Phone, Map, Navigation, WLAN, Bluetooth, Music, Video, and Messaging.

The application framework layer provides an application programming interface (application programming interface, API) and a programming framework for an application at the application layer. The application framework layer includes some predefined functions. As shown in FIG. 17, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, an address book, and the like.

The view system includes visual controls such as a control for displaying a text and a control for displaying an image. The view system may be used to construct an application. A display interface may include one or more views. For example, a display interface including an SMS message notification icon may include a text display view and an image display view.

The phone manager is configured to provide a communication function for the electronic device 100.

The resource manager provides various resources such as a localized character string, an icon, an image, a layout file, and a video file for an application.

The notification manager enables an application to display notification information in a status bar, and may be configured to convey a notification message. The notification manager may automatically disappear after a short pause without requiring a user interaction. For example, the notification manager is configured to notify download completion, give a message notification, and the like. The notification manager may alternatively be a notification that appears in a top status bar of the system in a form of a graph or a scroll bar text, for example, a notification of an application that is run on a background, or may be a notification that appears on the screen in a form of a dialog window. For example, text information is prompted in the status bar, an alert tone is made, the electronic device vibrates, or an indicator blinks.

The Android runtime includes a core library and a virtual machine. The Android runtime is responsible for scheduling and management of the Android system.

The core library includes two parts: a function that needs to be called in Java language and a kernel library of Android.

The application layer and the application framework layer run on the virtual machine. The virtual machine executes Java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (media library), a three-dimensional graphics processing library (for example, OpenGL ES), and a 2D graphics engine (for example, SGL). The surface manager is configured to manage a display subsystem and provide fusion of 2D and 3D layers for a plurality of applications. The media library supports play and recording in a plurality of commonly used audio and video formats, and static image files. The media library may support a plurality of audio and video encoding formats, for example, MPEG-4, H.264, MP3, AAC, AMR, JPG, and PNG. The three-dimensional graphics processing library is configured to implement three-dimensional graphics drawing, image rendering, composition, layer processing, and the like. The 2D graphics engine is a drawing engine for 2D drawing.

The kernel layer is a layer between hardware and software. The kernel layer includes at least a display driver, a camera driver, an audio driver, and a sensor driver.

The use of "one embodiment" or "an embodiment" in the specification means that particular features, structures, or characteristics described with reference to the embodiment are included in at least one example implementation solution or technology disclosed according to embodiments of this application. The phrase "in one embodiment" appearing in various places in the specification does not necessarily all mean a same embodiment.

The disclosure of embodiments of this application further relates to an apparatus for performing operations in the text. The apparatus may be constructed dedicatedly for the required purpose or may include a general-purpose computer that is selectively activated or reconfigured by a computer program stored in the computer. Such a computer program may be stored on a computer-readable medium, such as but not limited to, any type of disk, including a floppy disk, an optical disc, a CD-ROM, a magneto-optical disk, a read-only memory (ROM), a random access memory (RAM), an EPROM, an EEPROM, a magnetic or optical card, an application-specific integrated circuit (ASIC), and any type of medium suitable for storing electronic instructions. In addition, each of them may be coupled to a computer system bus. Moreover, the computer mentioned in the specification may include a single processor or may be an architecture using a plurality of processors for increased computing capabilities.

In addition, the language used in the specification is already mainly selected for readability and instructional purposes and may not be selected to depict or limit the disclosed topics. Therefore, embodiments of this application are intended to describe but not to limit the scope of the concepts discussed in the specification.

## Claims

1. An exercise control method, applied to a first electronic device, wherein the method comprises:

   obtaining an exercise target parameter of a user for an exercise apparatus;
   determining a first training plan of the user based on the exercise target parameter and a historical exercise capability parameter of the user, wherein the historical exercise capability parameter comprises a physical function parameter generated in a process in which the user takes exercise by using the exercise apparatus; and
   sending a first set of control instructions to the exercise apparatus based on the first training plan.

2. The method according to claim 1, wherein the first training plan comprises at least one action phase, and an exercise control parameter corresponding to each action phase matches the historical exercise capability parameter of the user.

3. The method according to claim 2, wherein the exercise control parameter corresponding to each action phase in the first training plan comprises a load parameter related to the exercise apparatus; and
   sending the first control instruction to the exercise apparatus based on the first training plan comprises:
   sending a first control instruction for a first action phase to the exercise apparatus based on a first load parameter corresponding to the first action phase, wherein the first control instruction comprises the first load parameter, and the first set of control instructions comprise the first control instruction.

4. The method according to claim 1, wherein the method further comprises:

   obtaining real-time physiological data currently collected by a second electronic device;
   adjusting the first training plan to a second training plan when detecting that the real-time physiological data meets a first trigger condition for adjusting a training plan, wherein at least one exercise control parameter in the first training plan is different from that in the second training plan; and
   sending a second set of control instructions to the exercise apparatus based on the second training plan.

5. The method according to claim 4, wherein the real-time physiological data comprises a real-time heart rate; and
   detecting that the real-time physiological data meets the first trigger condition for adjusting a training plan comprises:

   detecting that the real-time heart rate is less than a lower limit of a target heart rate zone; or
   detecting that the real-time heart rate is greater than an upper limit of a target heart rate zone.

6. The method according to claim 5, wherein adjusting the first training plan to the second training plan comprises:

when detecting that the real-time heart rate is less than the lower limit of the target heart rate zone, calculating, based on the lower limit, a load parameter increment corresponding to a next phase in the first training plan, and sending the load parameter increment to the exercise apparatus by using a second control instruction, wherein the second set of control instructions comprise the second control instruction; or
when detecting that the real-time heart rate is greater than the upper limit of the target heart rate zone, calculating, based on the upper limit, a load parameter decrement corresponding to a next phase in the first training plan, and sending the load parameter decrement to the exercise apparatus by using a third control instruction, wherein the second set of control instructions comprise the third control instruction.

7. The method according to claim 4, wherein before obtaining the real-time physiological data currently collected by the second electronic device, the method further comprises:

obtaining a current operating parameter of the exercise apparatus;
adjusting the first training plan to a third training plan based on a result of comparison between the current operating parameter and a target load parameter in a next phase in the first training plan, wherein at least one exercise control parameter in the first training plan is different from that in the third training plan; and
sending a third set of control instructions to the exercise apparatus based on the third training plan.

8. The method according to claim 7, wherein the exercise apparatus is a speed-type apparatus, the current operating parameter is a current speed, the target load parameter is a target speed, and the result of comparison between the current operating parameter and the target load parameter in the next phase in the first training plan comprises:

the current speed is less than the target speed; or
the current speed is greater than the target speed.

9. The method according to claim 8, wherein when the current speed is less than the target speed, adjusting the first training plan to the third training plan comprises any one of the following:

when detecting that a difference between the target speed and the current speed is less than a first difference threshold, sending the target speed to the exercise apparatus by using a fourth control instruction, to control a pace of the exercise apparatus to increase from the current speed to the target speed, wherein the third set of control instructions comprise the fourth control instruction;
when detecting that a difference between the target speed and the current speed is greater than or equal to the first difference threshold and is less than a second difference threshold, sending a difference between the target speed and a preset constant to the exercise apparatus by using a fifth control instruction, to control a pace of the exercise apparatus to increase from the current speed to the difference between the target speed and the preset constant, wherein the second difference threshold is greater than the first difference threshold, and the third set of control instructions comprise the fifth control instruction; or
when detecting that a difference between the target speed and the current speed is greater than a second difference threshold, obtaining the real-time physiological data currently collected by the second electronic device, and adjusting the first training plan based on the real-time physiological data.

10. The method according to claim 8, wherein when the current speed is greater than the target speed, adjusting the first training plan to the third training plan comprises any one of the following:

when detecting that a difference between the current speed and the target speed is less than or equal to a third difference threshold, sending the target speed to the exercise apparatus by using a sixth control instruction, to control a pace of the exercise apparatus to decrease from the current speed to the target speed, wherein the third set of control instructions comprise the sixth control instruction; or
when detecting that a difference between the current speed and the target speed is greater than a third difference threshold, obtaining the real-time physiological data currently collected by the second electronic device, and adjusting the first training plan based on the real-time physiological data.

11. The method according to claim 7, wherein the exercise apparatus is a resistance-type apparatus, the current operating parameter is a current power, the target load parameter is a target power, and the result of comparison between the current operating parameter and the target load parameter in the next phase in the first training plan comprises:

the current power is not equal to the target power.

12. The method according to claim 11, wherein when the current power is not equal to the target power, adjusting the first training plan to the third training plan comprises any one of the following:

when detecting that an absolute value of a difference between the current power and the target power is less than a fourth difference threshold, sending, to the exercise apparatus, a seventh control instruction instructing to maintain the current power for first preset duration;

when detecting that an absolute value of a difference between the current power and the target power is greater than or equal to the fourth difference threshold and is less than a fifth difference threshold, sending, to the exercise apparatus, an eighth control instruction instructing to maintain the current power for second preset duration, wherein the fifth difference threshold is greater than the fourth difference threshold; or

when detecting that an absolute value of a difference between the current power and the target power is greater than the fifth difference threshold, sending, to the exercise apparatus, an eighth control instruction instructing to maintain the current power for third preset duration, or obtaining the real-time physiological data currently collected by the second electronic device and adjusting the first training plan based on the real-time physiological data, wherein

the first preset duration is less than the second preset duration, and the second preset duration is less than the third preset duration.

13. An exercise control method, applied to a first electronic device, wherein the method comprises:

detecting an exercise target parameter input operation of a user for an exercise apparatus;

controlling, in response to the exercise target parameter input operation, the exercise apparatus to execute a corresponding first training plan; and

when detecting that an exercise status of the user meets a preset condition, controlling the exercise apparatus to execute a corresponding second training plan, wherein at least one operating parameter of the exercise apparatus in the corresponding first training plan is different from that in the corresponding second training plan.

14. An electronic device, comprising one or more processors and one or more memories, wherein the one or more memories store one or more programs, and when the one or more programs are executed by the one or more processors, the electronic device is enabled to perform the exercise control method according to any one of claims 1 to 13.

15. A computer-readable medium, wherein the readable medium stores instructions, and when the instructions are executed on a computer, the computer is enabled to perform the exercise control method according to any one of claims 1 to 13.

Steps

Heart rate

Blood oxygen saturation

Maximal oxygen uptake

20

XXX

XXX XXX XXX

**XXXX xx**
XXX XXX

Communication connection ~ TO FIG. 1B

Communication connection ~ TO FIG. 1C

FIG. 1A

CONT.
FROM
FIG. 1A

30

**Communication
connection**

TO
FIG. 1C

FIG. 1B

CONT.
FROM
FIG. 1A

CONT.
FROM
FIG. 1B

10

101

08:00

**Health**

102

XXXXX

XXXXXX    XXXX    XXXXXXX

XX    XX    XX    XX    XX

Exercise record
Aerobic endurance
running 30′02″
248 kcal burned

Start

XXXXXX

XXXX

XXXXXX

XXXX

XXXXXX

XXXX

**Health**  Exercise  Discovery  Device  Me

FIG. 1C

210

Aerobic endurance
running • 40 minutes

**Walk slowly for 4 minutes
at 4 kilometers per hour**

A watch X20 is connected
A treadmill X30 is connected

12

9

**00:00:35**

**4.0     0.03     3**
km/h     km     kcal

6

3

**110** ♥ Warm up ▐▐▐▐
Heart rate

0 (minutes)     7     14     21     28     35

FIG. 2

Compare the real-time physiological data with the exercise capability–related parameter provided by the capability model

FIG. 3

EP 4 620 538 A1

<u>100</u>

Antenna 1                                              Antenna 2

| Mobile communication module 2G/3G/4G/5G [150] | Wireless communication module BT/WLAN/GNSS/NFC/IR/FM [160] |

| Speaker [170A] | | | |
| Receiver [170B] | Audio module [170] | Processor [110] | Sensor module [180] |
| Microphone [170C] | | | Pressure sensor [180A] |
| Headset jack [170D] | | | Gyroscope sensor [180B] |
| Displays 1 to N [194] | | | Barometric pressure sensor [180C] |
| Cameras 1 to N [193] | | | Magnetic sensor [180D] |
| Indicator [192] | | | Acceleration sensor [180E] |
| Motor [191] | | | Distance sensor [180F] |
| Button [190] | | | Optical proximity sensor [180G] |
| SIM card interfaces 1 to N [195] | | | Fingerprint sensor [180H] |
| External memory interface [120] | | | Temperature sensor [180J] |
| Internal memory [121] | | | Touch sensor [180K] |

Ambient light sensor [180L]

Bone conduction sensor [180M]

| USB interface [130] | Charging management module [140] | Power management module [141] |

Charging input

Battery [142]

FIG. 4

200

FIG. 5

Watch 20

Mobile phone 10

Treadmill 30

601 Establish a running capability model of a user based on a historical exercise record of the user

602 Receive a training target set by the user

603 Obtain a running capability–related parameter provided by the running capability model

604 Generate a training plan that matches a running capability of the user

605 Send an exercise control parameter in each phase in the training plan →

606 Set a load based on the received exercise control parameter

Collect real-time physiological data and real-time exercise data of the user during exercise 607

608 Send the collected real-time physiological data and real-time exercise data →

Detect that an absolute value of a difference between the real-time exercise data and an interval corresponding to an exercise control parameter in a current phase is greater than a first threshold 609

TO
FIG. 6B

TO
FIG. 6B

TO
FIG. 6B

FIG. 6A

CONT.
FROM
FIG. 6A

CONT.
FROM
FIG. 6A

CONT.
FROM
FIG. 6A

610

Determine whether the absolute value of the difference between the real-time exercise data and the interval corresponding to the exercise control parameter in the current phase is greater than a second threshold

Yes

No

Adjust an exercise control parameter in a next phase in the training plan based on the real-time physiological data  611

612 Send an adjusted exercise control parameter in the next phase

613 Adjust a load in the next phase based on the adjusted exercise control parameter

614 Adjust exercise control parameters in the current phase and a next phase based on the real-time physiological data

615 Send adjusted exercise control parameters

616 Adjust loads in the current phase and in the next phase based on the adjusted exercise control parameters

FIG. 6B

FIG. 7a

<u>10</u>

720

08:00

**Indoor run**   Walk   Yoga   Fitness   Cy

721

**6.49**

Cumulative running (kilometers) >

722

Set target

723

724

Warm up

725

Music

726

**My running course**

🏃 **Heart rate-regulated running**
Make your own training plan

**Recommended**

🏃 Story run • Jungle rescue
L1 basic 22 minutes 202 kcal

Health   **Exercise**   Discovery   Device   Me

FIG. 7b

FIG. 8a

10

Course name

Heart rate-regulated running • Leisure-paced fast walking
L1 basic 3 kilometers 196 kcal

820

**Action phase**

821

▌ Warm up
Time 3 minutes
Heart rate zone [90, 100] bpm

822

▌ Slow walking
Distance 1 kilometer
Heart rate zone [100, 110] bpm

823

▌ Fast walking
Distance 1.5 kilometers
Heart rate zone [110, 120] bpm

Start

FIG. 8b

901 — Extract historical physiological data of a user based on a historical exercise record of the user

902 — Extract historical exercise data of the user based on the historical exercise record of the user

903 — Establish a running capability model of the user based on the historical physiological data and/or the historical exercise data of the user

904 — Determine, based on the running capability model of the user, a maximum speed interval that the user can bear

FIG. 9

1001 — Determine a current speed of a treadmill and a target speed in a next phase in a current training plan

1002 — Determine whether the current speed is less than the target speed

Yes

No

1003 — Calculate a first speed difference between the target speed and the current speed

1004 — Determine a result of comparison between the first speed difference and a preset speed difference interval, and determine a first adjustment policy for the current speed based on the result of comparison

1005 — Calculate a second speed difference between the current speed and the target speed

1006 — Determine a result of comparison between the second speed difference and a preset third threshold, and determine a second adjustment policy for the current speed based on the result of comparison

FIG. 10

1101

Obtain real-time heart rate data collected at each sampling moment in a current phase

1102

Determine whether a proportion of invalid data in the collected real-time heart rate data is greater than a preset abnormality proportion

No

1103

Determine whether an average real-time heart rate in the current phase is within a target heart rate zone

No

1104

Determine that the average real-time heart rate is less than a lower limit of the target heart rate zone, and calculate a target load in a next phase based on the lower limit

1105

Determine that the average real-time heart rate is greater than an upper limit of the target heart rate zone, and calculate the target load in the next phase based on the upper limit

Yes

Yes

1106

Do not need to adjust a training plan

FIG. 11

FIG. 12

Steps

Heart rate

Blood oxygen saturation

Maximal oxygen uptake

20

XXX

XXX XXX XXX

XXXX xx

XXX XXX

Communication connection

~ TO FIG. 13B

Communication connection

~ TO FIG. 13C

FIG. 13A

CONT.
FROM
FIG. 13A

40

Communication
connection

FIG. 13B

CONT.
FROM
FIG. 13A

CONT.
FROM
FIG. 13B

10

08:00

**Health**

XXXXX

XXXXXX     XXXX     XXXXXXX

XX     XX     XX     XX     XX

Exercise record

Spin bike 30′02″
248 kcal burned

Start

XXXXXX

XXXX

XXXXXX

XXXX

XXXXXX

XXXX

**Health**  Exercise  Discovery  Device  Me

FIG. 13C

Watch 20

Mobile
phone 10

Spin bike
40

1401  Establish a cycling capability model of a
user based on a historical exercise record of
the user

1402  Receive a training
target set by the user

1403  Obtain a cycling capability–related
parameter provided by the cycling
capability model

1404  Generate a training plan that
matches a cycling capability of
the user

1405
Send an exercise control
parameter in each phase
in the training plan

1406
Set a load based
on the received
exercise control
parameter

Collect real-time
physiological data  1407
and real-time
exercise data of the
user during exercise

1408
Send the collected real-
time physiological data
and real-time exercise data

Determine that an absolute value of
a difference between the real-time
exercise data and an interval
corresponding to an exercise control
parameter in a current phase is
greater than a fourth threshold

1409

TO
FIG. 14B

TO
FIG. 14B

TO
FIG. 14B

FIG. 14A

CONT.
FROM
FIG. 14A

CONT.
FROM
FIG. 14A

CONT.
FROM
FIG. 14A

1410

Determine
whether the absolute
value of the difference
between the real-time exercise
data and the interval corresponding to
the exercise control parameter in the
current phase is greater
than a fifth
threshold

Yes

No

Adjust an exercise control
parameter in a next phase in the
training plan based on the real-time
physiological data

1411

1412

Send an adjusted exercise
control parameter in the
next phase

1413

Adjust a load in the
next phase based on
the adjusted exercise
control parameter

1414

Adjust exercise control parameters
in the current phase and a next
phase based on the real-time
physiological data

1415

Send adjusted exercise
control parameters

1416

Adjust loads in the current
phase and the next phase
based on the adjusted
exercise control parameters

FIG. 14B

1501 — Determine a current power of a spin bike and a target power in a next phase in a current training plan

1502 — Determine whether the current power is equal to the target power

Yes

1503 — Calculate a power difference between the target power and the current power and take an absolute value

1504 — Determine a result of comparison between the absolute value of the power difference and a preset power difference interval, and determine an adjustment policy for the current power based on the result of comparison

No

1505 — Do not need to adjust a power load

FIG. 15

1601

Obtain real-time heart rate data collected at each sampling
moment in a current phase

1602

Determine whether
a proportion of invalid data in the
collected real-time heart rate data is
greater than a preset abnormality
proportion

No

1603

Determine
whether an average real-time heart rate in
the current phase is within a target
heart rate zone

No

1604

Determine that the average real-time heart rate is less than a
lower limit of the target heart rate zone, and calculate a target
load in a next phase based on a difference between the lower
limit and the average real-time heart rate

1605

Determine that the average real-time heart rate is greater than an
upper limit of the target heart rate zone, and calculate the target
load in the next phase based on a difference between the average
real-time heart rate and the upper limit

Yes

Yes

1606

Do not need to adjust a training plan

FIG. 16

| Application layer | Health | Calendar | Map | WLAN | Music | Messaging |
|---|---|---|---|---|---|---|
| | Gallery | Phone | Navigation | Bluetooth | Video | ... |

| Application framework layer | Window manager | Content provider | Phone manager | Resource manager |
|---|---|---|---|---|
| | Notification manager | View system | ... | |

| System library | Surface manager | Three-dimensional graphics processing library | Android runtime |
|---|---|---|---|
| | 2D graphics engine | Media library | ... |

| Kernel layer | Display driver | Camera driver | Touch driver |
|---|---|---|---|
| | Audio driver | Sensor driver | ... |

FIG. 17

## INTERNATIONAL SEARCH REPORT

| International application No. |
| --- |
| **PCT/CN2024/073070** |

| A. | CLASSIFICATION OF SUBJECT MATTER |
| --- | --- |

A63B24/00(2006.01)i; G06F16/9035(2019.01)i

According to International Patent Classification (IPC) or to both national classification and IPC

| B. | FIELDS SEARCHED |
| --- | --- |

Minimum documentation searched (classification system followed by classification symbols)

   IPC:A63B,G06F16

Documentation searched other than minimum documentation to the extent that such documents are included in the fields searched

Electronic data base consulted during the international search (name of data base and, where practicable, search terms used)

   CNTXT, ENTXTC, VEN, CNKI: 运动, 控制, 指导, 目标, 参数, 训练, 计划, 心率, 功率, 载荷, 阻力, motion, control+, guid+, target, parameter, training, plan, heart, rate, power, load, resistance

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 116936027 A (GUANGDONG OPPO MOBILE TELECOMMUNICATIONS CORP. LTD.) 24 October 2023 (2023-10-24)<br>description, specific embodiments, and claims 1-13 | 1-15 |
| X | CN 109300521 A (BEIJING CALORIE INFORMATION TECHNOLOGY CO., LTD.) 01 February 2019 (2019-02-01)<br>description, specific embodiments, and claims 1-13 | 1-15 |
| X | CN 106709235 A (FENGPAO SPORTS DEVELOPMENT (SHENZHEN) CO., LTD.) 24 May 2017 (2017-05-24)<br>description, specific embodiments | 1-15 |
| X | CN 103007493 A (NIKE INC.) 03 April 2013 (2013-04-03)<br>claims 1-3 | 1 |
| A | CN 113948183 A (HUAWEI TECHNOLOGIES CO., LTD.) 18 January 2022 (2022-01-18)<br>entire document | 1-15 |
| A | CN 114783562 A (HISENSE GROUP HOLDINGS CO., LTD.) 22 July 2022 (2022-07-22)<br>entire document | 1-15 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **24 April 2024** | **28 April 2024** |

| Name and mailing address of the ISA/CN | Authorized officer |
| --- | --- |
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/073070** |

| C. | DOCUMENTS CONSIDERED TO BE RELEVANT | |
|---|---|---|
| Category* | Citation of document, with indication, where appropriate, of the relevant passages | Relevant to claim No. |
| A | US 10456077 B1 (CHUANG THOMAS CHU SHAN) 29 October 2019 (2019-10-29) entire document | 1-15 |
| A | US 2017333755 A1 (KUAIWEAR LTD.) 23 November 2017 (2017-11-23) entire document | 1-15 |

Form PCT/ISA/210 (second sheet) (July 2022)

**INTERNATIONAL SEARCH REPORT**
Information on patent family members

International application No.

**PCT/CN2024/073070**

| Patent document cited in search report | | | Publication date (day/month/year) | Patent family member(s) | | | Publication date (day/month/year) |
|---|---|---|---|---|---|---|---|
| CN | 116936027 | A | 24 October 2023 | None | | | |
| CN | 109300521 | A | 01 February 2019 | None | | | |
| CN | 106709235 | A | 24 May 2017 | None | | | |
| CN | 103007493 | A | 03 April 2013 | WO | 2008030484 | A2 | 13 March 2008 |
| | | | | WO | 2008030484 | A3 | 10 July 2008 |
| | | | | EP | 2059310 | A2 | 20 May 2009 |
| CN | 113948183 | A | 18 January 2022 | None | | | |
| CN | 114783562 | A | 22 July 2022 | None | | | |
| US | 10456077 | B1 | 29 October 2019 | None | | | |
| US | 2017333755 | A1 | 23 November 2017 | US | 2017333754 | A1 | 23 November 2017 |

Form PCT/ISA/210 (patent family annex) (July 2022)

**EP 4 620 538 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310377258